(19) Europäisches Patentamt
European Patent Office
Office européen des brevets

(11) EP 3 368 014 B1

(12) EUROPEAN PATENT SPECIFICATION

(45) Date of publication and mention of the grant of the patent:
**04.10.2023 Bulletin 2023/40**

(21) Application number: **16798004.4**

(22) Date of filing: **26.10.2016**

(51) International Patent Classification (IPC):
***A61K 9/127*** *(2006.01)* ***A61K 9/51*** *(2006.01)*
***A61K 31/436*** *(2006.01)*

(52) Cooperative Patent Classification (CPC):
(C-Sets available)
**A61K 31/352; A61K 9/127; A61K 9/5123; A61K 31/05; A61K 36/185; A61K 45/06** (Cont.)

(86) International application number:
**PCT/IL2016/051154**

(87) International publication number:
**WO 2017/072762 (04.05.2017 Gazette 2017/18)**

# (54) NOVEL CANNABINOID FORMULATIONS

NEUARTIGE CANNABINOIDFORMULIERUNGEN

NOUVELLES FORMULATIONS DE CANNABINOÏDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2015 US 201562246224 P**

(43) Date of publication of application:
**05.09.2018 Bulletin 2018/36**

(73) Proprietor: **Yissum Research Development Company of the Hebrew University of Jerusalem Ltd**
**9139002 Jerusalem (IL)**

(72) Inventors:
- **DOMB, Abraham J.**
**Jerusalem 9359006 (IL)**
- **HOFFMAN, Amnon**
**9378422 Jerusalem (IL)**
- **IZGELOV, Dvora**
**5845121 Holon (IL)**

(74) Representative: **Zacco GmbH**
**Bayerstrasse 83**
**80335 München (DE)**

(56) References cited:
**WO-A1-2013/108254 WO-A1-2016/144376**

Remarks:
The file contains technical information submitted after the application was filed and not included in this specification

EP 3 368 014 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/05, A61K 2300/00;**
**A61K 31/352, A61K 2300/00**

## Description

### TECHNOLOGICAL FIELD

The invention generally relates to formulations for oral administration of cannabinoids, particularly cannabinoid combinations.

### BACKGROUND

The notion of synergism between various cannabinoids stems from clinical experience with medicinal cannabis. Any type of a natural cannabis plant contains over 60 types of cannabinoids. A series of clinical studies suggested that extracts of natural cannabis are more efficacious and less hazardous than their synthetic or purified cannabinoid constituents alone, such as Tetrahydrocannabinol (THC) or Cannabidiol (CBD). Patients with Multiple Sclerosis (MS), for example, reported preference of natural cannabis to Marinol® (dronabinol, THC, a synthetic form of the main THC isomer, trans-$\Delta^9$-THC). Further, studies in-vivo suggested that the levels of THC in the brain are elevated in the presence of CBD. Two controlled double-blind clinical trials examining the effect of THC:CBD 1:1 combination on muscle spasms (337 subjects) and central neuropathic pain (66 subjects) in MS showed significant improvement of these symptoms in the treated group vs. placebo. In contrast, an analogous study of Marinol effects on central/peripheral neuropathic pain found no significant clinical benefits, and further reported significantly poorer tolerance to Marinol adverse effects. Phase III study of patients with intractable and opioid non-responsive cancer pain reported that the analgesic efficacy of THC:CBD 1:1 combination was better than THC-based product. These and many other studies pointed at specific clinical advantages of cannabinoid combinations, particularly those comprising THC and CBD in equal and other proportions.

The interest in CBD has been prompted by early findings of its alleviating effects on THC-related anxiety and panic, both in animals and humans. More recently, it has been demonstrated that CBD acts on THC via a pharmacokinetic mechanism, specifically by inhibition of THC metabolic enzymes, and more specifically by reducing the formation of 11-hydroxy-THC, one of the psychoactive THC metabolites.

Although the therapeutic rationale behind delivery of THC:CBD combinations has been acknowledged, achieving therapeutically effective formulations of a combination of these two actives and modes of effective oral delivery thereof still remain to be resolved. This is in view of apparent failure of the existing delivery systems to provide high, uniform and reproducible bioavailability of both actives simultaneously. There are a number of reasons for poor oral bioavailability of THC and CBD. Both are highly lipophilic and therefore are poorly soluble ex-vivo and in-vivo._Certain presystemic events at the gastro intestinal (GI) tract and first pass metabolism, mostly at pre-entrocytes and intra-enterocyte levels, also contribute to their poor bioavailability.

One example of a recent commercialized THC:CBD product is Sativex® approved in a number of countries across Europe, Canada, New Zealand and Israel for spastisity and neuropatic pain in MS, and in Canada for pain in cancer. Sativex, however, is liquid and administered by spray onto the oromucosal surface to bypass first pass metabolism, which limits its use especially when used chronically. Specifically, Sativex formulation contains excipients that often lead to lesions, ulcerations, pain and soreness of the oral mucosa, leading to interruption of treatment. This adds to pharmacokinetic data for Sativex showing notable inter-subject variability after single and repeated dosing. These variable and erratic individual profiles have been translated to increased daily administrations, nearly by 3-fold on average. Compliance of patients to such frequent daily drug regimen is usually poor, partially because of the ensuing economic burden.

Previous disclosures in this field generally related to formulations of lipophilic agents: US 7,919,113 which describes formulations of lipophilic agents forming a microemulsion; WO07056242 which describes certain self-emulsifying drug delivery systems; and WO13108254 which describes nano lipospheres-based formulations incorporating piperine.

### GENERAL DESCRIPTION

Any references in the description to methods of treatment refer to the products of the present invention for use in a method of treatment.

The instant invention addresses the above referred to need for effective oral delivery of cannabinoids and cannabinoid combinations, while maximizing their specific therapeutic effects, obtaining stable and predictable plasma drug levels, and therefore obtaining reproducible pharmacodynamic effects, and ultimately increasing patients' drug compliance.

Disclosed herein are formulations and methods for improved oral delivery of cannabinoids, and particularly cannabinoid combinations, with proven value in terms of stability, bioavailability and safety. Validity of the present inventive concept has been exemplified for compositions comprising two cannabinoids that are essentially distinct by

their physical, chemical and biological (therapeutic) properties, such as THC:CBD combinations. This implies that the same approach can be applied to other types of cannabinoids and cannabinoid-based actives, i.e., cannabinoid isoforms, derivatives, precursors or metabolites, including natural and synthetic cannabinoids. Furthermore, this approach forms the basis for providing formulations and methods applicable to combinations of cannabinoids and other therapeutic agents, drugs and food supplements, which until now have been considered incompatible in terms of solubility, stability and oral bioavailability. Being flexible and modulatory, formulations and methods are further relevant to personalized clinical applications, whereby improved stability, bioavailability and pharmacodynamic effect can lead to reduction of actives' therapeutic dose.

Further exemplified are liquid formulations, essentially composed of a combination of surfactants, $C_8$-$C_{12}$ fatty lipids and organic solvents specifically adapted to contain cannabinoid compositions in a single dosage form. One prominent feature of the above formulations is that upon contact with an aqueous medium, e.g., upon oral administration and contact with body fluids, such as those present in the GI tract, these formulations form nanoparticles that disperse in the GI fluids, thereby increasing in vivo bioavailability. This is without jeopardizing their tolerability and safety in humans.

Due to this particular feature, formulations of the invention are also referred to herein as pro-nano lipospheres ('***PNL***'), particles or droplets.

It should be appreciated that the exact mechanism of action of formulations of the invention in the gut is unknown. It is equally possible that the observed improved bioavailability of these formulations can be attributed to particles per se, or to interaction(s) between therein embedded actives and other constituents, or both.

This present achievement with an effective oral delivery of THC:CBD combinations should be appreciated in view of the aforementioned limitations of the available THC:CBD products. These two actives, due to their specific physical, chemical properties, are known for being poorly soluble in body fluids, having limited ability to cross biological membranes, significant P-glycoprotein (P-gp) transport effect and first pass metabolism, and exceptionally poor oral bioavailability. The presently developed formulations and methods overcome these limitations in making these two actives available in the blood circulation, or in dissolving and dispersing thereof to the extent they cross biological membranes, or in protecting thereof from being metabolized or reverse transported.

Further, this achievement is moreover surprising in view of the recognized lability of THC and CBD, in particular, to oxidation, hydrolysis, and thermal and photolytic degradation in ambient light and temperature, which impose significant limitations on obtaining successful formulations of THC and CBD, in both liquid and solid forms. Degradation of THC (a strong psychoactive cannabinoid) leads to the formation of cannabinol (CBN (a weak psychoactive cannabinoid). Moreover, THC and CBD, and other cannabinoids, have tendency to adhere to solid surfaces.

The present achievement should further be appreciated in view of desirability of THC:CBD containing products and methods of effective delivery thereof. Potentiation of THC related therapeutic effects and reduction of THC adverse effects by CBD has been previously mentioned. By virtue of acting in distinct biological pathways, the effects of these two cannabinoids are further complementary. While THC, or $\Delta^9$-THC, is a partial agonist of the $CB_1$ (neuronal) and $CB_2$ (immune) receptors, CBD displays high potency as an antagonist of $CB_1$/$CB_2$ receptors. This underlies the differential psychotropic effects of THC and CBD, as well as their differential effects on immunological and inflammatory processes, and thus their different clinical uses.

## BRIEF DESCRIPTION OF DRAWINGS

Fig. 1 shows plasma CBD concentration vs. time plot (mean ± SEM) following per os (PO) administration to mice of CBD (♦) and CBD formulation of the invention, CBD-PNL(▲) with CBD dose 15 mg/kg (n=6 in each group).

**Figs. 2A-2C** provide a comparative study of plasma CBD concentration vs. time plot (mean±SEM) following PO administering to mice of various CBD formulations with CBD dose 15 mg/kg, an absorption enhancers dose 10 mg/kg (n=6 in each group). **Fig. 2A** shows CBD (♦), CBD-PNL (▲) and CBD-Curcumin-PNL (x). **Fig. 2B** shows CBD (♦), CBD-PNL (▲) and CBD-Resveratrol-PNL (■). **Fig. 2C** shows CBD (♦), CBD-PNL (▲), CBD-piperine-PNL (■) and CBD with piperine in solution (x).

**Fig. 3** shows an analogous study with various THC formulations. Plasma THC concentration vs time plot (mean ± SEM) is shown for THC (▲), THC-PNL (♦), THC-piperine-PNL (■), and THC with piperine in solution (x), with THC 20 mg/kg, piperrine 10 mg/kg.

**Figs. 4A-4D** show results of a preliminary clinical study in healthy volunteers receiving a single dose various formulations of the inventions vs. Sativex, while monitoring for blood THC and CBD concentrations (mean ± SEM) under fasting over 24 h period. **Fig. 4A-4B** show blood THC and CBD levels for piperine-PNL THC:CBD formulation of the invention, P-PNL-THC:CBD (■) with THC dose of approximately 20 mg, and CBD - 20 mg CBD compared to Sativex (♦) (n=3). **Fig. 4C-4D** show blood THC and CBD levels for piperine-PNL formulations containing THC or CBD with approximately 10 mg actives, P-PNL-THC or P-PNL-CBD (♦), respectively, compared to Sativex (■) (n=9).

**Fig. 5** shows results of Phase 1, randomized, clinical study (n=15) for piperine-PNL, P-PNL:THC:CBD (■), PNL-THC:CBD (x) formulations of the invention and Sativex (●), relating to mean blood CBD concentrations.

**Fig. 6** shows results of the same trial, P-PNL:THC:CBD (■), PNL-THC:CBD (x) formulations of the invention and Sativex (●), relating to mean blood THC concentrations

**Fig. 7** shows results of the same trial, P-PNL:THC:CBD (■), PNL-THC:CBD (x) formulations of the invention and Sativex (●), relating to mean blood 11-OH-THC concentrations, the main active THC metabolite.

## DETAILED DESCRIPTION OF EMBODIMENTS

The scope of the invention is defined by the claims.

The invention provides a formulation for use in oral administering of an effective amount of a cannabinoid composition comprising a combination of Tetrahydrocannabinol (THC) and Cannabidiol (CBD), or an isoform, a derivative, a precursor, or a metabolite thereof selected from Δ9 -THC, Δ8 -THC, an acid form of THC or CBD, namely THC-A or CBD-A, 11-OH-THC and THC-11-oic acid forms, in pharmaceutically acceptable carrier or excipient, the cannabinoid composition comprising between 0.2 and 15wt% of THC and CBD of the total weight of the formulation, the formulation further comprising (a) at least one surfactant, (b) at least one lipid component, (c) a water soluble biocompatible amphiphilic solvent, wherein the formulation is free of piperine; and wherein upon administering and contact with an aqueous medium, the formulation is converted into particles of a size of less than about 500 nm, thereby increasing bioavailability of the administered cannabinoid composition.

In one example, not being part of the invention, is provided a method for oral administration of a cannabinoid composition, the method comprising

**(i)** administering to a subject a formulation comprising

- **(a)** at least one surfactant,
- **(b)** at least one lipid component,
- **(c)** a water soluble biocompatible amphiphilic solvent, and

**(ii)** administering to said subject a therapeutically effective amount of a cannabinoid composition comprising a combination of cannabinoids, cannabinoid isoforms, derivatives, precursors or metabolites in pharmaceutically acceptable carrier or excipient,

such that upon administration and contact with an aqueous medium, the formulation is converted into, particles of a size of less than about 500 nm, thereby increasing bioavailability of the administered cannabinoid composition.

In certain embodiments, in the above method the formulation in **(i)** and the cannabinoid composition in **(ii)** are administered simultaneously or in succession.

In some embodiments, the methods apply to oral administration of the formulation in (i) and Sativex for improving oral bioavailability of the latter.

In another example, not being part of the invention, is provided a method for oral administration of a cannabinoid composition, whereby in accordance to said method a subject is administered with a formulation comprising

- **(a)** a therapeutically effective amount of a cannabinoid composition comprising a combination of cannabinoids, cannabinoid isoforms, derivatives, precursors or metabolites,
- **(b)** at least one surfactant,
- **(c)** at least one lipid component,
- **(d)** a water soluble biocompatible amphiphilic solvent,

such that upon administration, the formulation is converted into particles of a size of less than about 500 nm, thereby increasing bioavailability of the cannabinoid composition.

In some embodiments, formulations used in the above methods form particles of less than about 200 nm, less than about 100 nm or less than about 50 nm, and further particles in the range of at least about 10-100 nm, 20-90 nm, 30-80 nm, 40-70 nm or 50-60 nm, or in the range of at least about 10-30 nm.

In some embodiments, particles size is selected from about 10-500 nm, 10-400 nm, 10-300 nm, 10-200 nm, and 10-100 nm.

In other embodiments, particles size is selected from about 10-300 nm, 10-250 nm, 10-200 nm, 10-150 nm, 10-100 nm, and 10-50 nm.

In still other embodiments, particles size is selected from about 10-100 nm, 10-90 nm, 10-80 nm, 10-70 nm, 10-60 nm, 10-50 nm, 10-40 nm, 10-30 nm, 10-20 nm.

In some embodiments, particles size is in the range of approximately 10-200 nm, 10-190 nm, 10-180 nm, 10-170 nm, 10-160 nm, 10-150 nm, 10-140 nm, 10-130 nm, 10-120 nm, 10-110 nm, 10-100 nm, 10-90 nm, 10-80 nm, 10-70 nm, 10-60 nm, 10-50 nm, 10-40 nm, 10-30 nm, or 10-20 nm.

In some embodiments, the particles size is about 10, 20, 30, 40, 50, 60, 70, 80, 90 or 100 nm.

In some embodiments of any one or more of the formulation or methods disclosed herein, the particles size is between about 10 and 200 nm.

Determination of particles' diameter, polydispersity index, and an average ζ potential can be carried out by means of a number of known methods in the art. Examples of such measurements are provided herein, e.g., in EXAMPLE 2 and **Table 1.**

As used herein, the term '***cannabinoid*',** or any lingual variation thereof, encompasses the class of chemical compounds, cannabinoid/cannabinoid agonists/cannabinoid-related compounds, acting with various affinities on the endogenous cannabinoid receptors (CB 1 and CB2). This group include the endocannabinoids (produced naturally by humans and animals), phytocannabinoids (found in cannabis and some other plants), and synthetic cannabinoids (manufactured artificially), the most notable of which are THC and CBD.

In some cases, the term is made in reference to the classical cannabinoids originating from, or mimicking, the natural cannabinoids produced by cannabis plants. Over 80 different cannabinoids have been isolated from various strains of cannabis, so far. The main classes of the classical cannabinoids are listed below.

| Type | Structure |
|---|---|
| Cannabigerol-type **CBG** | 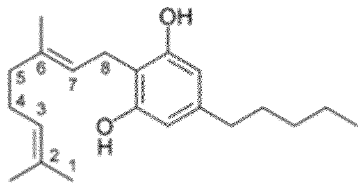 |
| Cannabichromene-type **CBC** | 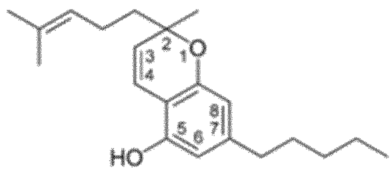 |
| Cannabidiol-type **CBD** | 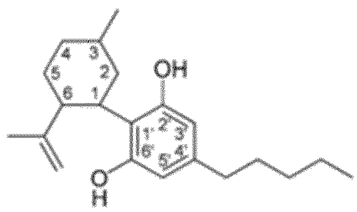 |
| Tetrahydrocannabinol-and Cannabinol-type **THC, CBN** | 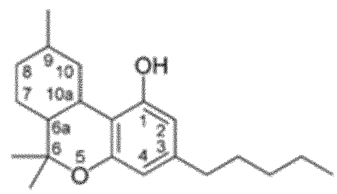 |
| Cannabielsoin-type **CBE** | 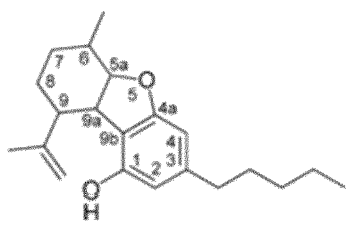 |

(continued)

| Type | Structure |
|---|---|
| iso-Tetrahydrocannabinol-type ***iso*-THC** | 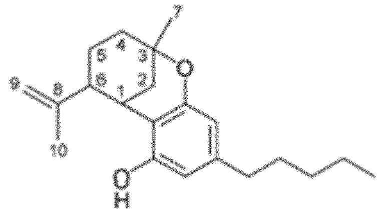 |
| Cannabicyclol-type **CBL** | 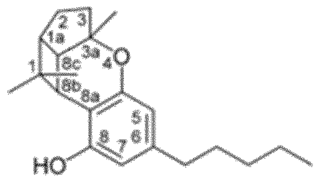 |
| Cannabicitran-type **CBT** | 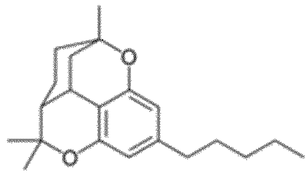 |

The cannabinoid composition of the invention comprises a combination of tetrahydrocannabinol (THC) and Cannabidiol (CBD), or an isoform, a derivative, a precursor, or a metabolite thereof selected from Δ9-THC, Δ8-THC, an acid form of THC or CBD, namely THC-A or CBD-A, 11-OH-THC and THC-11-oic acid forms.

The formulations as referred to herein, unless otherwise specified, refer to formulations of the invention, e.g., PNL-THC:CBD and P-PNL-THC:CBD formulations.

Chemical structures of THC and CBD are shown below.

***THC***

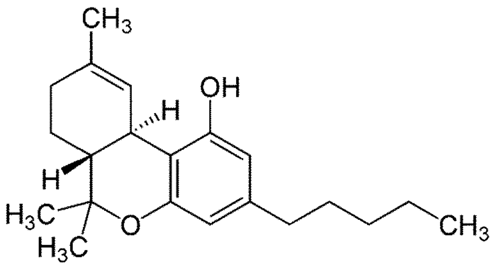

***CBD***

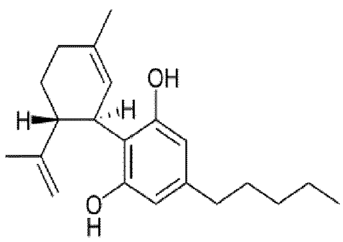

All classes of cannabinoids derived from cannabigerol-type compounds and differ mainly in the way this precursor is cyclized. The classical cannabinoids are derived from their respective 2-carboxylic acids (2-COOH, also denoted with -A) by decarboxylation (catalyzed by heat, light, or alkaline conditions). Of particular relevance to the invention are the THC and CBD acid precursors, THC-A and CBD-A.

In one embodiment, the cannabinoid composition comprises a combination of THC and CBD or an isoform, a derivative, a precursor, or a metabolite thereof as claimed that is a synthetic cannabinoid or a natural cannabinoid obtained from cannabis extracts as single cannabinoid or a combination of cannabinoids.

In some embodiments, formulations used in accordance with the invention comprise natural cannabinoids in a form of a single or combination of cannabis extracts. The term '***natural cannabis extract***' pertains to any type of extract from any type of cannabis plant, e.g., oils extract, tinctures or alcohol extractions, etc., of a cannabis plant or a plant material or part thereof, such as flowers, e.g., flowers of female plants.

In some embodiments, methods apply to oral formulations comprising combinations of THC:CBD (as the actives), having actives content of between 0.2 and 15% THC and CBD (per weight), or more, or between 0.2-1%, or 1-5%, 5-10%, or 10-15% THC and CBD, and combinations of these proportions.

Methods can be further articulated as methods of using or administering oral formulations comprising THC:CBD combinations with the ratio of THC:CBD is between 0.01:99.99 and 99.99:0.01 (per weight).

In certain embodiments, methods can use or administer formulations comprising THC:CBD combination wherein the ratio THC:CBD is approximately 1:1, proven as other combinations to be advantageous for the treatment or alleviation of many disorders.

In yet other embodiments, methods use formulations wherein THC is in excess. Such methods are particularly applicable, for example, in the context of treatment of chronic pain, weight loss or appetite loss due to AIDS, and for nausea caused by cancer chemotherapy, as well as to many other clinical applications.

In still other embodiments, methods use formulations wherein CBD is in excess. CBD is known for its analgesic, anti-inflammatory and neuroprotective properties.

It should be appreciated that methods apply to a wide range of THC:CBD and other cannabinoid compositions. An important feature of the methods is their capability to improve the bioavailability of cannabinoid compositions and their ensuing wide clinical applicability. Anorexia, emesis, pain, inflammation, multiple sclerosis, neurodegenerative disorders (such as Parkinson's disease, Huntington's disease, Tourette's syndrome, Alzheimer's disease), epilepsy, autism, fibromyalgia, tuberculosis, inflammatory bowel diseases, including ulcerative colitis and Crohn's disease, irritable bowel syndrome, glaucoma, osteoporosis, schizophrenia, cardiovascular disorders, cancer, obesity, and metabolic syndrome-related disorders, is only a partial list of clinical conditions that are treatable by methods and formulations of the invention.

The **'*improved bioavailability*'** refers to the bioavailability achieved with formulations of the invention as compared to bioavailability achieved and measured for other cannabinoid compositions, such depicted, e.g., as in **Fig. 1** to **Fig. 7** herein. Improved bioavailability of formulations and methods have been presently exemplified in relation to Sativex, a cannabinoid formulation in clinical trial and the golden standard for cannabinoid treatments.

In certain embodiments, bioavailability of the present formulations compared to one of the known oral cannabinoid compositions can be in the range of about 5-10%, 5-15%, 5-20%, and further above 30, 40, 50, 60, 70, 80, 90, 100% and more.

Due to their generality and wide clinical applicability, in some embodiments, methods apply to formulations or compositions comprising additional therapeutically active or non-active agents, or combinations thereof, being part of the cannabinoid formulation, or administered independently simultaneously or in succession thereto. In some embodiments, these are therapeutic non-active agents for improving bioavailability of actives, such as absorption enhancers.

It should be noted that according to the invention absorption enhancers are not obligatory constituents to ensure improved bioavailability of cannabinoids, as has been presently demonstrated in a human clinical trial.

Notwithstanding, certain absorption enhancers can be desirable for the purpose of certain embodiments and applications, whether being part of formulations of the invention or administered in conjunction therewith. Among presently tested agents, curcumin, resveratrol and piperine.

A method for oral administration of a cannabinoid composition is further described (not being part of the invention), whereby according to said method a subject is administered with a gelatin capsule comprising a formulation that comprises

**(a)** a therapeutically effective amount of a cannabinoid composition or isoforms, derivatives, precursors or metabolites thereof,
**(b)** at least one surfactant,
**(c)** at least one lipid component,
**(d)** a water soluble biocompatible amphiphilic solvent, and
**(e)** optionally an absorption enhancer,

such that upon administration or contact with an aqueous medium the formulation is converted into particles of a size of less than about 500 nm, thereby increasing bioavailability of the cannabinoid composition.

It is further meant that for the purpose of numerous embodiments and applications, formulations of the invention can comprise at least one additional therapeutically active agent, whether being part of the formulation or being administered in conjunction therewith. Under this particular feature is conceived any therapeutic agent, drug, or a combination of drugs, from one or more General Drug Categories classified by the FDA according to their clinical effects and applicability to human disorders, and also vitamins and food supplements, such as omega fatty acids, omega-3-fatty acids (EPA, DHA, ALA). In some embodiments, there are therapeutically active agents selected from at least one group of analgesics, antirheumatic, antibiotics, chemotherapeutic drugs, immunosuppresants, antipsychotics, drugs for neurological disorders, drugs for the treatment of AIDS, and combinations thereof.

In some embodiments, the therapeutic agents are selected amongst antibiotics, anti-epileptics, anti-spastics, anti-inflammatory drugs, analgesics and antipsychotic drugs.

The presently described methods can be further applicable to include additional poorly water soluble drugs. Examples of such drugs in the context of cannabinoid-based therapies include, although not limited to: analgesic/antirheumatic group (Ibuprofen, Diclofenac), from antibiotics/ chemothepeutic group (Nitrofurantoin), and drugs for the treatment

of AIDS (Nevirapine).

It should be noted that in the case of certain cannabinoids or cannabinoid-extracts, formulations used in accordance with the invention can further comprise taste masking agents such as sweeteners, flavors, or peppermint oil.

The term ***'surfactant'*** as meant herein encompasses amphiphilic compounds selected from anionic, cationic, nonionic, and zwitterionic compounds. The surfactant may be selected from polyoxyethylene, sorbitan monolaurate, sorbitan monooleate and mixtures thereof.

In some embodiments, formulations can comprise at least one surfactant that is at least one high HLB (hydrophilic/lipophilic balance) surfactant with HLB of at least about 8, and at least one low HLB surfactant with HLB of less than about 5.

The term ***'lipid component'*** herein encompasses solid or liquid water insoluble materials that are soluble in the formulation. Non-limiting examples of such solid components include fatty acids, hydrogenated vegetable oils, fatty amines and fatty alcohols or their respective esters or amides that are solids as room temperatures (25-27°C; in other words, the solids have a melting point that is above 25-27°C), polymers that are solids at room temperatures, and paraffins and waxes that are solids at room temperatures. Liquid components include natural oils (olive, sesame, sunflower, linseed oil, hemp and coconut oils), medium chain fats and liquid paraffin.

In some embodiments, formulations comprise at least one lipid component that is a fatty acid, a fatty amine, a fatty alcohol or a fatty ester or a mixture thereof.

Under the term ***'lipid component'*** is further meant solid lipid components such as tricaprin, trilaurin, trimyristine, tripalmitin, tristearin and mixtures thereof that are solids at 25-27°C, and further compounds that solidify *in situ* upon dispersion in aqueous medium, e.g., partially or fully hydrogenated vegetable oil that are solid at 25-27°C.

In some embodiments, formulations comprise at least one lipid component that is a mono-, a di-, a triglyceride, a fatty acid ester with long and short chain alcohols, tricaprin, trilaurin, trimyristine, tripalmitin, tristearin or a mixture thereof.

The term ***'fatty acid esters'*** herein refers to mono-, di-, and triglycerides and fatty acid esters with long and short chain alcohols that are solids at room temperature.

In some embodiments, formulations comprise an ethoxylated fat and fatty compounds.

The terms '***ethoxylated fat'*** and ***'fatty compound*** are further meant to encompass polyethyleneglycol-hydrogenated castor oils, e.g., cremophor and cremophor RH, and further phospholipids, e.g., an egg phospholipid, a soy phospholipid and lecithin of various grades and purities.

In certain embodiments, formulations comprise or further comprise a phospholipid, or a combination of phospholipids.

In further embodiments, the formulations comprise at least one phospholipid that is selected from an egg phospholipid, a soy phospholipid and lecithin of various grades and purities.

The term '***amphiphilic solvent'*** refers to compounds selected from lower alkyl (having between 1 and 8 carbon atoms) esters of lactic acid, lower alkyl (having between 1 and 8 carbon atoms) lactone esters and N-methylpyrrolidone. Some non-limiting examples of lower alkyl esters include methyl, ethyl, propyl, isopropyl, butyl, hexyl, pentyl and octyl esters.

In some embodiments, the amphiphilic solvent is selected from methyl lactate, ethyl lactate, propyl lactate, spironolactone and N-methylpyrrolidone.

In other embodiments, the amphiphilic solvent is a combination of a lower alkyl ester of lactic acid with N-methylpyrrolidone.

In some embodiments, the amphiphilic solvent comprises a combination of a solvents selected from the family of lower alkyl esters of lactic acid together with a solvent selected from the family of alkyl lactone esters or N-methylpyrrolidone.

In other embodiments, the amphiphilic solvent is combined with a hydrophilic organic solvent such as ethylene glycol, glycofurol or PEG 400.

In some embodiments, the formulation can be dispersed in water with a ratio of approximately 1:5 (formulation:water) or more, and further in the range of 1:5, 1:10, 1:20, 1:30, 1:40, 1:50, and up to 1:100 ratio, respectively, or more.

One particularly advantageous feature of the formulations is that they can be loaded into a pressure forming capsule for oral delivery. Thus, a gelatin capsule is also exemplified herein, comprising a formulation as disclosed herein (in respect of any formulation of the invention), such a formulation, for example, consisting of comprising of a plurality of particles of a size of less than about 500 nm, wherein the nanoparticles comprise:

**(a)** a cannabinoid composition comprising a combination of cannabinoids, cannabinoid isoforms, derivatives, precursors or metabolites as claimed,
**(b)** at least one surfactant,
**(c)** at least one lipid component, and
**(d)** a water soluble biocompatible amphiphilic solvent.

In some embodiments, formulations used in the above methods form particles of a size as disclosed herein.

In certain embodiments, the formulations can be compressed into tablets or pills for oral administering.

It should be appreciated that in some embodiments the formulation can further comprise at least one of an antioxidant, an absorption enhancer, a color- and a flavor-imparting agent, a preservative, a stabilizer, a salt, or a combination thereof.

Various sweeteners, taste modifiers, antioxidants, preservatives which are well known in the art can serve these purposes. For example, taste modifiers such as artificial sweeteners, flavorings as strawberry and peppermint oil, for example, and further plant sweeteners, sugars, honey, citrate, acids, menthol; anise, eucalyptus oil, fennel, antioxidants such as vitamins E (tocopherol) and C and their derivatives, butylated hydroxyanisole (BHA), butylated hydroxytolune (BHT) recognized as GRAS, and sulfides, and ascorbyle palmitate; any sweetener allowed for oral administration such as sugar, glucose, sucralose, cyclamate, sucrose, saccharin, fructose, maltose, stevia extract, sodium saccharine; salts such as NaCl, $NaHCO_3$, $Na_2CO_3$, citrate, and others.

Other additives may be used, such as nutrients, vitamins, various solidifiers and viscosity modifiers, such as stearic acid, cetyl acid, polymers, cetyl alcohol, cetostearyl alcohols, stearyl alcohol; and specific viscosity enhancers such as alginate, PG alginate, Carbopol, mucoadhesive polymers, Carbophils, celluloses, Pluronics and Pluronic F127.

In certain embodiments formulations can be further adapted to facilitate gastro-resistance and/or controlled release of the active ingredients or drugs. The term ***'controlled release'*** refers to a coating or a modification enabling to achieve time dependent release, sustained release, prolonged release, and further pulse release, or delayed release of the actives or drugs. Under the term ***'gastro-resistance'*** is meant a coating or a modification enabling to achieve pH-controlled drug release, gastrointestinal targeting, colon delivery, protection of acid-sensitive actives, protection of gastric mucosa from aggressive actives. In this sense, gastro-resistance is also targeted drug release. Gastro-resistant coatings and modifications are especially applicable to oral dose formulations. They can also increase drug effectiveness and provide improved storage stability.

Gastro-resistance and/or controlled release may be achieved in the case of solid formulations, after adsorption of the formulation to a substrate, by modification of coating using for example poly(meth)acrylates coating or layering. One example of poly(meth)acrylate coating widely used in the pharmacological industry to achieve targeted and controlled drug release is EUDRAGIT®.

It should be appreciated that formulations can form particles of a size of less than about 200 nm, less than about 100 nm or less than about 50 nm, and further particles in the range of at least about 10-100 nm, 20-90 nm, 30-80 nm, 40-70 nm and 50-60 nm, and of at least about 10-30 nm.

The formulations comprise a combination of THC and CBD, or isoforms, derivatives, precursors, metabolites thereof, as claimed. Advantageousness of such compositions has been previously discussed along with difficulties to achieve successful dissolution and stability of the actives. The present success with THC:CBD formulations is moreover surprising in view of apparently unsuccessful trials with analogous agents such as paclitaxel, amphotericin B, bupivacaine free base, triamcinolone and dexamethasone, and cyclosporine A in particular. These presently developed THC and CBD formulations were further related to improved stability, bioavailability and safety in human clinical trials.

Thus in some embodiments, is provided formulations comprising between 0.2 and 15% of a combination of THC and CBD (per weight).

In certain embodiments, formulations comprise between 0.2 and 10% wt of a combination of THC and CBD actives.

In some embodiments, the amount of active combination is 0.2, 0.3, 0.4, 0.5, 0.6, 0.7, 0.8, 0.9, 1, 2, 3, 4, 5, 6, 7, 8, 9, 10, 11, 12, 13, 14 and 15% of the total weight of the formulation. In certain embodiments, the amount of actives combination is between 0.2 and 9%, between 0.2 and 8%, between 0.2 and 7%, between 0.2 and 6%, between 0.2 and 6%, between 0.2 and 5%, between 0.2 and 4%, between 0.2 and 3%, between 0.2 and 2%, between 0.2 and 1.5% or between 0.2 and 1%.

In further embodiments, the amount of actives is between 1-15%, 1-14%, 1-13%, 1-12%, 1-11%, 1-10%, 1-9%, 1-8%, 1-7%, 1-6%, 1-5%, 1-4%, 1-3% or between 1-2% of the total weight of the formulation.

In some embodiments, the ratio of THC:CBD in the formulations is between 0.01:99.99 and 99.99:0.01.

In certain embodiments, the ratio between THC:CBD is between 0.05:99.95 and 99.95:0.05, between 0.1:99.9 and 99.9:0.1, between 1:99 and 99:1, between 2:98 and 98:2, between 3:97 and 97:3, between 5:95 and 95:5, between 10:90 and 90:10, between 15:85 and 85:15, between 20:80 and 80:20, between 25:75 and 75:25, between 30:70 and 70:30, between 35:65 and 65:35, between 40:60 and 60:40, or between 55:45 and 45:55.

In further embodiments, THC is present in excess. More specifically, the ratio THC:CBD is 99.99:0.01, 99.95:0.05, 99.9:0.1, 99:1, 98:2, 97:3, 95:5, 90:10, 85:15, 80:20, 75:25, 70:30, 65:35 or 60:40.

In other embodiments, CBD is present in excess. In some embodiments, the ratio THC:CBD is 0.01:99.99, 0.05:99.95, 0.1:99.9, 1:99, 2:98, 3:97, 5:95, 10:90, 15:85, 20:80, 25:75, 30:70, 35:65 or 40:60.

In yet further embodiments, the ratio THC:CBD in the formulations of the invention is approximately 1:1. The term '***approximately'*** herein refers to a deviation of at least about 10% (+/-) in the measured content (wt).

In certain embodiments, the formulation comprises between 0.2 and 10% actives, the actives being THC and CBD at a ratio of about 1:1.

In some embodiments, formulations according to the above comprise a 1:1 ratio of THC:CBD at a total amount of 0.2, 0.3, 0.4, 0.5, 0.6, 0.7, 0.8, 0.9, 1, 2, 3, 4, 5, 6, 7, 8, 9 or 10% wt of the total weight of the formulation. In some embodiments, the amount is between 0.2 and 9%, between 0.2 and 8%, between 0.2 and 7%, between 0.2 and 6%, between 0.2 and 6%, between 0.2 and 5%, between 0.2 and 4%, between 0.2 and 3%, between 0.2 and 2%, between 0.2 and 1.5% or between 0.2 and 1%. In some embodiments, the amount is between 1 and 10%, between 1 and 9%, between 1 and 8%, between 1 and 7%, between 1 and 6%, between 1 and 5%, between 1 and 4%, between 1 and 3% or between 1 and 2%.

Of particular relevance are compositions of THC and CBD compositing at least one of $\Delta^9$-THC, $\Delta^8$-THC, THC-A, CBD-A or 11-OH-THC, THC-11-oic acid forms.

The at least one cannabinoid may be a synthetic, a semi-synthetic or a natural cannabinoid. In some embodiments, the combination of cannabinoids comprise natural cannabinoid in a form of a single or a combination of cannabis extracts.

The the formulations are free of piperine.

In some embodiments, the formulations further comprise at least one additional poorly water soluble therapeutically active agent, or a combination thereof.

The used herein, the ***'poorly water soluble drug'*** is a liquid drugs, certain non-limiting examples thereof include: albendazole, danazole, ketoconazole, itrconazole, atovaquone, troglitazone, valsartan, nimesulide, loratadine, griseofulvin, felodipine, probucol, ubiquinone, cefixime, frusemide, salicylic acid, ketoprofen, tinidazole, aceclofenac, hydrocholthiazide, ofloxacin, ibuprofen, nevirapine.

In some embodiments, the poorly soluble drug is selected amongst hormones, supplements, vitamins, or combinations thereof. Examples of poorly water soluble vitamins include vitamins A, D, E and K.

In another example (not being part of the invention) is disclosed a kit comprising:

**(a)** a formulation comprising at least one surfactant, at least one lipid, a water soluble biocompatible amphiphilic solvent and water, such that the formulation is convertible, upon contact with a water medium, into particles of a size of less than about 500 nm, or less than about 200 nm, 100 nm or 50 nm or less.
**(b)** a cannabinoid composition comprising a combination of cannabinoids, cannabinoid isoforms, derivatives, precursors or metabolites in a carrier or an excipient,
**(c)** instructions for use.

In some embodiemnts, **(b)** can be a cannabinoid fromulation of the invention in water or water with a taste masking agent and/or antioxidant.

The kit is intended for achieving a controlled therapeutic effect, wherein each of the multiple components of the kit may be administered simultaneously or each of said multiple dosage forms may be administered sequentially in either order.

More specifically, the kit includes containers for separate compositions, such that **(a)** and **(b)** are in separate containers, vials, bottles or a divided packet. In certain embodiments, separate compositions may also be contained within a single, undivided container. Typically the kit includes directions for the administration of separate components.

In some embodiments, therapeutically effective amount of the formulations comprising cannabinoid compositions **(b)** and/or formulation without cannabinoids **(a)** are personalized to achieve a personalized dose. The term ***'personalized dose'*** refers to a method wherein the therapeutically effective dose is tailored to the individual patient based on their predicted response and alleviation of symptoms of a disease. In this method, diagnostic testing may be employed for selecting appropriate and optimal therapies based on the context of a patient's molecular or biochemical analyses and other (potentially genetic).

In certain embodiments, the kit can further comprise at least one additional poorly water soluble therapeutically active agent, or a combination thereof.

Also exemplified and not being part of the invention is a method for achieving a personalized therapeutically effective amount of at least one poorly water soluble therapeutic agent in a subject treated therewith, the method comprising administering to the subject:

**(i)** an amount of a formulation comprising

**(a)** at least one surfactant,
**(b)** at least one lipid component,
**(c)** a water soluble biocompatible amphiphilic solvent, and

**(ii)** an amount of at least one poorly water soluble therapeutic agent or a formulation comprising the same and **(a), (b)** and **(c),**

such that upon administering the formulations in **(i)** and in **(ii)** are converted into particles of a size of less than about 500 nm, thereby increasing bioavailability of said poorly water soluble therapeutic agent,

**(iii)** monitoring of at least one therapeutic effect in the subject to adjust the amount of the formulation in **(i)** and/or the amount of the poorly water soluble therapeutic agent or the formulation in **(ii)** to achieve a personalized therapeutically effective amount of the poorly water soluble therapeutic agent or the formulation thereof to be administered to the subject.

The term ***'therapeutically effective amount'*** or ***'dose'*** means an amount of an active related to any change in a treated condition as measured by the relevant definition criteria, i.e., therapeutic effect, either in an animal model or in a clinical trial. In this sense, the therapeutic effect is also a pharmacodymanic effect. The term ***'personalized therapeutically effective amount'*** further refers to an individual therapeutic dosage after repeated trials and optimization.

In certain embodiments, a change in the condition being treated is identified if there is at least 5% improvement, or 10% improvement, or at least 25%, or at least 50%, or at least 75%, or at least 100% improvement. The change can be based on improvements in the severity of the treated condition in an individual, or on a difference in the frequency of improved conditions in populations of subjects with and without treatment with the dosage forms of the invention, or with the dosage forms of the invention in combination with other drugs.

A therapeutically effective amount (also pharmacologically or pharmaceutically or physiologically effective amount) means herein the amount of active agent (a cannabinoid or a combination) in a pharmaceutical composition that is needed to provide a desired level of active agent in the bloodstream or at a target organ of the subject, to provide an anticipated physiological response. The precise amount will depend upon numerous factors, e.g. type of an agent, activity of a composition, intended patient use (e.g. number of doses per day), patient considerations, and others, which can readily be determined by one skilled in the art. An effective amount of an agent can be administered in one administration, or through multiple administrations of an amount that total an effective amount, preferably within a 24-hour period. It can be determined using standard clinical procedures for determining appropriate amounts and timing of administration. It is understood that the effective amount can be the result of empirical and/or individualized (case-by-case) determination on the part of the treating health care professional and/or individual.

The term 'therapeutically effective amount' further refers to the amount of the actives (as a combination or a single component) in terms of mg per body weight per day, further depending on the number of administrations per day. This amount can be in the range of at least about 1 to 100 mg active(s) per kg body weight per day, e.g., 1-10, 1-20, 1-30, 1-40, 1-50, 1-60, 1-70, 1-80, 1-90 and 1-100 mg/Kg/day, or more.

In some embodiments, the effective amount is at least about 1 to 1,000 μg active(s) per kg body weight per day, e.g., 1-10, 1-20, 1-30, 1-40, 1-50, 1-60, 1-70, 1-80, 1-90 and 1-100 μg/Kg/day, or more.

In some embodiments, the effective amount is below 1,000 μg active(s) per kg body weight per day

The above method can be further applied to the presently developed formulations, wherein under at least one poorly water soluble therapeutic agent is meant a cannabinoid composition comprising a combination of cannabinoids, cannabinoid isoforms, derivatives, precursors or metabolites in pharmaceutically acceptable carrier or excipient.

In an aspect, the invention provides a formulation for use in oral administering of an effective amount of a cannabinoid composition comprising a combination of Tetrahydrocannabinol (THC) and Cannabidiol (CBD), or an isoform, a derivative, a precursor, or a metabolite thereof selected from $\Delta^9$-THC, $\Delta^8$-THC, an acid form of THC or CBD, namely THC-A or CBD-A, 11-OH-THC and THC-11-oic acid forms, in pharmaceutically acceptable carrier or excipient, the cannabinoid composition comprising between 0.2 and 15wt% of THC and CBD of the total weight of the formulation, the formulation further comprising

**(a)** at least one surfactant,
**(b)** at least one lipid component,
**(c)** a water soluble biocompatible amphiphilic solvent,

wherein the formulation is free of piperine; and

such that upon administering and contact with an aqueous medium, the formulation is converted into particles of a size of less than about 500 nm, thereby increasing bioavailability of the administered cannabinoid composition.

In certain embodiments, the formulation according to the above is adapted for being administered simultaneously with the cannabinoid composition or in succession.

In numerous embodiments, said formulation is adapted for being administered simultaneously with the cannabinoid composition or in succession.

It should be appreciated that the disclosure further provides a process for preparing a formulation, said process comprising:

**(I)** dissolving an amphiphilic solvent and optionally a phospholipid,
**(II)** adding at least one surfactant to the solution obtained in step (I) to obtain a homogenous solution;
**(III)** adding a pre-determined amount of a combination of THC and CBD at a desired ratio to the pre-concentrate formed in step **(II)** to obtain a homogenous solution which upon contact with an aqueous phase, spontaneously forms drug (THC/CBD) encapsulated nano-dispersion.

## WORKING EXAMPLES

***Reagents and methods***

Chemicals used in the present experiments are listed below:

THC as Dronabinol was obtained from THC PHARM;
CBD - from STI Pharmaceuticals Ltd;
Piperine, curcumin and resveratrol - from SABINA CORPORATION;
Polysorbate 20 (tween 20) - from Merk KGaA;
Sorbitan monooleate (span 80) - from OFER Chem. Lab. Suppliers;
Lechitins - from Cargill;
Tricaprine - from CREMER Oleo Division;
Polyoxyl 40 Hydrogenated Castor Oil (HCO 40) - from BASF Chem. Comp.;
Ethyl lactate - from PURAC.

***Characterization of PNL-cannabinoid formulations***

Particle size and ζ potential were determined using Zetasizer Nano ZS ZEN 3600 (Malvern Instruments Ltd, Malvern, UK). Prior to determination of particle size and ζ potential, 200 μl of the pre-concentrate were vortex-mixed in 1800 μl distilled water at 37° C for 30s forming a dilution in a ratio of 1:10 (v/v). Measurements were taken using Folded Capillary Cells (Malvern Instruments Ltd, Malvern,UK). Prior to measurements, the cell was flushed with ethanol and de-ionised water.

***Studies in animals***

Male Wistar rats (Harlan, Israel), 300-350 g were used in the experimental procedures. The study was approved by the Animal Experimentation Ethics Committee of the Hebrew University Hadassah Medical School Jerusalem (IACUC).

PNL and Advanced-PNLs (incorporating curcumin, resveratrol or piperine) were freshly prepared 30 min before each experiment, by vortex-mixing of the pre-concentrates in pre-heated to 37C° water (1:10 v/v) for 30 sec forming an O/W nano-dispersion. The cannabinoid concentration was 3mg/mL. PNL formulations were administered to animals by oral gavage (n=6). One control group received cannabinoid composition other than PNL at the same concentration (n=6). Another control group received solution (propylene glycol:ethanol:water, 4.5:4.5:1). The administered dose of cannabinoid in the experimental and first control groups was 15 mg/kg.

Animals were anesthetized for the period of surgery by intra-peritoneal injection of 1 mL/kg of ketamine-xylazine solution (9:1 respectively), placed on a heated surface and maintained at 37°C (Harvard Apparatus Inc., Holliston, MA). An indwelling cannula was placed in the right jugular vein of each animal for systemic blood sampling using previously described methods. The cannula was tunneled beneath the skin and exteriorized at the dorsal part of the neck. After completion of the surgical procedure, the animals were transferred to cages to recover overnight (12-18 h). During this recovery period, food, but not water, was deprived. Throughout the experiment free access to food was available 4h post oral administration. Animals were randomly assigned to the different experimental groups.

***Studies in humans***

A preliminary clinical study included healthy volunteers receiving a single dose of various formulations of the inventions vs. Sativex as eight actuations, while monitoring for blood THC and CBD concentrations (mean ± SEM) under fasting over 24h period. Subjects received piperine-PNL THC:CBD formulation, P-PNL-THC:CBD, comprising THC:CBD dose of approximately 20 mg each compared to Sativex (n=3), or piperine-PNL formulations containing THC or CBD only, P-PNL-THC or P-PNL-CBD, comprising approximately 10 mg actives each, compared to Sativex (n=9).

The study was approved by the Animal Experimentation Ethics Committee of the Hebrew University Hadassah Medical School Jerusalem (IACUC).

Phase 1, single-center, open-label, randomized, comparative 5-period, 5-way crossover single-dose study included 15 healthy male volunteers admitted and monitored at the clinical research center (CRC) 12 h before each dosing session. Subjects received a standardized morning meal, within 30 min a single dose of PNL-THC:CBD, P-PNL-THC:CBD (Advanced PNL containing piperine) or Sativex was administered. Subjects remained at the CRC and were monitored over 24 h period. Blood samples were periodically drawn for pharmacokinetics (PK) analyses and vital signs and safety parameters were recorded. Subjects remained confined in bed, or in a sitting position, until a meal was served 4 h after drug administration. Subjects were discharged from the ward 24 h after dosing after approval of the study physician. Subjects were enforced a minimum 4-day wash-out period between each treatment session. An end-of-the-study (EOS) visit was conducted 7-10 days after the last treatment session. Subjects were selected according to rigorous predefined inclusion and exclusion criteria. The study protocol and informed consent forms (ICFs) were approved by the Tel Aviv Sourasky Medical Center Institutional Review Board (IRB), and by the Clinical Trials Department of the Ministry of Health.

Tested products included: ***PNL-THC:CBD*** gelatin oral capsules (3.6 mg THC and 3.3 mg CBD per capsule), total dose 10.8 mg THC and 10 mg CBD; and ***P-PNL-THC:CBD*** gelatin oral capsules (3.6 mg THC, 3.3 mg CBD and 10 mg piperine per capsule), total dose 7.2 mg THC, 6.7 mg CBD and 20 mg piperine. The reference product was: ***Sativex oromucosal spray,*** 4 actuations, two under the tongue and 2 inside the cheek, each actuation contained 2.7 mg THC and 2.5 mg CBD, total dose: 10.8mg THC and 10mg CBD.

Blood samples were drawn within 60 min of dosing, and 0.5, 1, 1.5, 2, 2.5, 3, 3.5, 4, 5, 8, 12 and 24 h post-dosing were used to determine THC, 11-OH-THC and/or CBD $C_{max}$, $T_{max}$, $AUC_T$, $AUC_{Inf}$, and $T_{½}$. Pharmacokinetic parameters were determined for THC, 11-OH-THC and CBD. A linear mixed effects analysis was performed on $C_{max}$, $T_{max}$, $AUC_T$, with fixed terms for sequence, mode of administration, period, and a random term for volunteer nested within sequence using log-transformed pharmacokinetic parameters. For each parameter estimates and 90% confidence intervals for the geometric means between the all relevant pairs of administration ways were obtained by computing estimates and 90% confidence intervals for the difference between the different ways of administration means within the mixed model framework. All statistical analyses were performed using SAS v9.3 or higher (SAS Institute, Cary NC, USA).

For evaluation of safety, adverse events were recorded throughout the study period. Vital signs were monitored throughout the 24-h in-house sessions and at the end of the study visit. A physical examination was performed before each dosing session, prior to discharge from the clinical research center and at the end of study visit. A 12-lead ECG and safety laboratory evaluations were performed at the end of study visit.

## EXAMPLE 1

### *1.1 Preparation of PNL and Advanced-PNL (P-PNL) formulations*

Excipients used for preparing blank pro-nanoparticulates are listed below:

Polysorbate 20 (tween 20) - 14.1% (w/w);
Sorbitan monooleate (span 80) - 14.1
Lechitin - 8.3%
Tricaprine - 14.1%;
HCO 40 - 14.1%;
Ethyl lactate - 35.4%.

PNL cannabinoid formulations were prepared by pro-nanoparticulate method. The final PNL composition was determined in optimization studies according to optimal solubilization capacity of actives and smallest particle size obtained upon dilution of the pre-concentrate in aqueous phase. An amphiphilic co-solvent (ethyl lactate) and soy phospholipid (4:1) respectively were placed in a clean scintillation tube and heated to 40°C until complete dissolution. A triglyceride (tricaprin), polyoxyl 40-hydroxy castor oil, Tween 20, and Span 80 were added (ratios of 1:1:1:1). The mixture was gently stirred and heated to 40°C till homogenous solution was formed.

For animal studies, the active ingredient(s) was added, forming CBD-PNL pre-concentrate containing CBD 3%(w/w) or THC-PNL containing THC 3%(w/w), gently stirred and heated to 40°C till homogenous solution was formed. Upon gentle agitation in aqueous phase, these pre-concentrates spontaneously form drug encapsulated O/W nano-dispersion. An absorption enhancer was incorporated into PNL in order to form Advanced-PNL by the following method. A polyphenol (curcumin or resveratrol) or an alkaloid (piperine) was added to the CBD-PNL pre-concentrate to form three Advanced-PNLs; CBD-Curcumin-PNL, CBD-Piperine-PNL and CBD-Resveratrol-PNL. Additionally, Piperine was

added to THC-PNL to form THC-Piperine-PNL. The amount of each absorption enhancer in each of the Advanced-PNLs was 2%(w/w).

*__1.2 Evaluation of drug load__*

For animal and in-vitro studies, loading of actives in various PNL-cannabinoid formulations, i.e., CBD-PNL, THC-PNL was assessed following 1:10 dilutions of the pre-concentrates prepared as described above. The obtained solution (400μL) was placed into Nanosep® centrifugal devices (Pall Life Sciences, Ann Arbor, MI, US) with 30K cut-off membranes and centrifuged for 30 min. at 10,000 RPM at 25°C. Additionally, CBD and THC solution (300μg/mL) was placed inside similar centrifugal devices and centrifuged in the same manner to assess the non-specific adsorption of CBD and THC to the test-tube and membrane surface. Duplicates were used for each concentration..

The non-specific adsorption (NSA) percent of THC or CBD was calculated as:

$$100 - \frac{\text{CBD or THC solution conc. after centrifugation } * 100}{\mathit{CBD\ or\ THC}\text{ solution conc. before centrifugation}}$$

THC or CBD load percent was calculated as:

$$100 - \frac{\text{CBD solution conc. after centrifugation of CBD} - \text{PNL } * 100}{(\mathit{CBD}\text{ solution conc. before centrifugation of CBD} - \text{PNL}) * (100\% - \text{NSA}\%)}$$

To determine the ability of the formulation to form nanoparticles loaded with the drug, base formulation was loaded into an automatic syringe that released into water a stream of base formulation for one hour. The released formulation dispersed immediately to form an almost clear solution which showed >90% transmission when examined in a UV/VIS at 550 nm for transmission.

**EXAMPLE 2**

*__2.1 Characterization of PNL and Advanced PNL in vitro__*

PNL and Advanced-PNL (P-PNL) pre-concentrates were dispersed in water (1:10, v/v) prior to their administration to animals. The mean particles diameter, polydispersity index, and an average ζ potential are presented in **Table 1.**

**Table 1:** Particle Size, ζ Potential and Polydispersity Index (PDI) of various PNLs and Advanced-PNLs Obtained by 1:10 v/v dilution in Aqueous Phase. (Data presented as mean ± SD, n=3).

| Formulation | Size nm (diameter) | Zeta potential mV | *PdI |
|---|---|---|---|
| **CBD-PNL** | 26 | -13 | 0.23 |
| **THC-PNL** | 30 | -12 | 0.22 |
| **CBD-piperine-PNL** | 30 | -15 | 0.2 |
| **CBD-resveratrol-PNL** | 65 | -10 | 0.5 |
| **THC-piperine-PNL** | 40 | -12.5 | 0.25 |

CBD-PNL, THC-PNL, CBD-Piperine-PNL, CBD-Resveratrol-PNL and THC-Piperine-PNL, were assessed for drug load dispersion in aqueous phase resulted in high drug load, both for CBD and THC, of >99% of the drug initially dissolved in the pre-concentrate. These results indicate that the amount of free drug, i.e. CBD and THC, not incorporated into these delivery systems was negligible.

2.2 *__Studies of combinatory PNL formulations__*

A series of optimization studies were carried out:

***2.2.1*** 1 ml blank pro-nanoparticulate formulation used 100 mg of cyclosporine was dissolved to form uniform clear solution, which upon the addition of a drop of the formulation yielded formation of nanoparticles of about 40 nm. To this cyclosporine solution, 50 and 100 mg of paclitaxel, amphotericin B, bupivacaine free base, CBD, triamcinolone and dexamethasone were added and allowed to dissolve. The mixtures were examined visually for complete dissolution, precipitation after 24 hours at refrigeration and for particle size when added to water. The addition of THC affected the cyclosporine original formulation with a pronounced effect for the 100 mg formulations. The paclitaxel and amphotericin B did not fully dissolve in the original formulation and precipitated-out after refrigeration.

Addition of CBD at 50 mg formed clear solution in the cyclosporine solution; however, an increase in the amount of CBD caused precipitation after a few days, at room temperature. The particle size of the formulations, when added to water, was above 100 nm, compared to the original formulation, as determined by transmission UV at 550 nm turbidity test.

This experiment demonstrated that mixing two or more active agents in the formulation affected the quality of the formulation and limited its use in the delivery of two agents in a single solution. Extensive testing and adjustments were required to design a formulation that could fit two or more agents in a single formulation. It is surprising that THC and CBD could form a stable solution at a concentration of 10% w/v that did not precipitate and form a suitable particle size below 100 nm when added to water.

***2.2.2*** The tricaprin component, in the base formulation, was replaced with liquid medium chain triglycerides or sesame oil to form a clear oily solution. 10% w/v of total THC and CBD were dissolved in this solution at any ratio to form clear solutions. When mixed in water a clear dispersion was obtained with almost full transmission when tested by UV/VIS spectrophotometer at 550nm which indicate a fine nano-dispersion. This experiment demonstrated that liquid oil is suitable for making the pro-lipid nano-dispersion for CBD and THC.

***2.2.3*** 10% w/w clear solution of THC and CBD in the pro-nanodispersion formulation was absorbed in porous polymeric or silica microparticles. The following particles were tested for absorption of the formulation: Poly-Pore E200 (INCI: allyl methacrylate crosslinked polymer, highly porous particles), Neusilin® US2 a fine ultralight granule of magnesium aluminometasilicate and widely accepted as a multifuntional excipient that improves the quality of pharmaceuticals (FUJI chemical Industry); silica gel, porous polystyrene beads (200-500 micron) with 0.5% crosslinking.

1 gram of a base formulation was mixed with increasing amounts of the absorbents until a free flowing powder was obtained. 150, 250, 300 and 350 mg of Poly-Pore, Neusilin® US2, polystyrene beads and silica gel absorbed 1 ml of the formulation to form a free flowing powder, respectively. The powders were easily loaded in hard gelatin capsules with gentle press, remaining stable without any oil leaking out of the capsules when placed horizontal onto filter paper for one week at room temperature. Poly-pore powder loaded with formulation base at a ratio of 1:2 was mixed with mixrocrystalline cellulose powder at a 1:1 ratio and granulated with PVP and the formed granules were compressed into tablets that remain stable. This experiment was repeated with 5% CBD solution in the base formulation with similar results.

Polypore powders loaded with base formulation at a 1:4 w/v ratio were dispersed in water with agitation to determine the release from the particles and nanoparticle formation. Nanoparticles were formed over one hour period which indicated that loading into porous particles can provide a controlled release effect in the GI tract. When 5% CBD formulation was loaded into Polypore at a 1:2 ratio and dispersed in excess water pH 2 at 37°C, CBD was released to the water for over 2 hours as determined by UV absorption. The aqueous media remained almost clear which indicate nanoparticles formation.

***2.2.4*** 13 molecules, some are common drugs of diverse chemical and physical properties have been mixed in the blank Liposphere formulation and in the 5% w/V CBD loaded formulation. In a typical experiment, 5 mg of the second agent was added to 95 mg of CBD solution containing 5 mg CBD in a base formulation mixture (clear solution off ethyl lactate, Tween 80, Span 20, tricaprin, phospholipid and Cremophor RH40). Heat was used in cases where the agent did not dissolve at room temperature. The mixtures were evaluated after one hour at room temperature and after refrigeration for 6 and 13 days. **Table 2** summarizes the stability and particle size data for the formulation after preparation and after 13 days. Out of the tested 13 molecules, four did not dissolve in the blank as well as in the CBD loaded formulation, two interfered with the CBD solution and precipitated. In three formulations, the added second molecule increased the particle size to over 60 nm after dispersion in water and only four agents did not alter the particle size of the dispersed formulation.

**Table 2:** Stability and particle size of various combinatory PNL formulations: X indicates no solubility, V indicates solubility.

| | **Solubility in** | **3hr after dissolution** | **after 13 days in 4C** | | | | | |
|---|---|---|---|---|---|---|---|---|
| Material (5%) | PNL | PNL with 5% CBD | Particle size* | PDI | Visual | Particle size* | PDI | Visual |
| **Cholesterol** | **V** | **V** | **96.43** | 0.487** | turbid | 86.94 | 0.516 | turbid |
| **Amphitericin B** | **X** | **X** | **-** | - | - | - | - | - |
| **Cyclosporine** | **V** | **V** | **31.74** | 0.102 | clear | 31.82 | 0.134 | clear |
| **Dexamethasone** | **V** | **V** | **313.8** | >1*** | milky | 253.3 | >1 | milky |
| **Methylprednisolone** | **V** | **V** | **22.59** | 0.206 | clear | 21.58 | 0.16 | clear |
| **Deoxycholic acid** | **V** | **V** | **41.6** | 0.577 | clear | 20.83 | 0.417 | clear |
| **Cholesteryl stearate** | **X** | **X** | **-** | - | - | - | - | - |
| **Cholic acid** | **X** | **X** | **-** | - | - | - | - | - |
| **Chloramphenicol** | **V** | **V** | **30.03** | 0.118 | clear | 29.64 | 0.172 | clear |
| **5,5-diethylbarbituric acid** | **X** | **X** | **-** | - | - | - | - | - |
| **Ibuprofen** | **V** | **V** | **74.28** | 0.398 | clear | 67.86 | 0.367 | clear |
| **Benzocaine** | **V** | **V** | **62.22** | 0.449 | clear | 65.82 | 0.479 | clear |
| **Paclitxel** | **V** | ~ | ~**50** | | clear | precipit ate | | |
| **THC-CBD-PNL (5%,5%)** | **V** | **V** | **38.49** | 0.263 | clear | ~30 | ~0.2 | clear |

This experiment indicates that there is no predictable process to determine which compound will co-dissolve in CBD without changing the particle size. Thus the combination of CBD/THC to form a 5/5 %w/w in the base formulation and remain sable is surprising, particularly when this formulation already confirmed stability for over 3 months.

Formulations prepared by mixing the dry solid powder of the tested compound in either the blank formulation to form 5% w/v or in a formulation already containing 5% w/w CBD. Particle size was determined by particle size analyzer.

*2.3* ***Optimization of drug load***

For Phase 1 clinical trial pro-nanoparticulate (PNL) formulation was prepared according to the above. PNL was added to the pre-weighted CBD. Bottles were kept in $37^0$C until complete dissolution of the CBD. CBD-PNL was diluted (in a 1:9 ratio) with water pre-heated to $37^0$C. Solutions were tested for particle size and visual inspection as detailed in **Table 3.**

**Table 3:** Preliminary screening for maximum CBD loading.

| % CBD | CBD (mg) | PNL (uL) | particle size (nm) | Pdi | visual inspection |
|---|---|---|---|---|---|
| blank | | 1000 | 23.12 | 0.191 | clear |
| 10% | 100 | 900 | 33.6 | 0.198 | clear |
| 12.50% | 125 | 875 | 42.85 | 0.281 | turbid |
| 15% | 150 | 850 | 62.1 | 0.384 | turbid |
| 20% | 200 | 800 | 171.5 | 0.458 | milky |

The maximum CBD load allowing formation of clear solution was 10-12.5%; a turbid nanoemulsion formed above these concentrations.

Four CBD-PNL formulations at concentrations of 6, 7, 8 and 10% were kept in room temperature and refrigerated storage conditions, protected from light. All preparations were done on a weight basis. Each formulation was tested for appearance, particle size and distribution. Duplicate tests were performed at manufacturing date and after 1, 2 and 3 months. Analytical test for CBD content was conducted using LC-MS.

Particle size of the preparations remained approximately the same in the course of two months under both temperatures. At the 3-months' time point, there was a slight increase in particle size at 2-8°C.

Distribution of size (poly dispersity) was below 0.4 at all-time points and storage conditions. After 3 months of storage at $2\text{-}8^0$C, a decrease in poly dispersity was observed.

Preparations kept in room temperature were in the form of a clear yellow liquid with an oily texture. Upon dilution with water, a clear solution was formed in all time points. Preparations kept in the refrigerator were solidified. Once exposed to room temperature, they became liquid as those in the room temperature. Clear solution was obtained upon dilution in all time points.

***2.4 Stability studies of dispersed and non-dispersed formulation***

***2.4.1*** Physical stability as measured by particle size of the PNL-THC:CBD and P-PNL-THC:CBD formulations of the invention was estimated under various conditions, at 2-8°C, at the room temperature (RT) and at 40°C (**Table 4**). These results support storage at 2-8°C or room temperature and 40 °C with no effect on the ability to form nanoparticles of less than 50 nm.

**Table 4:** Physical stability by particle size of PNL-THC:CBD and P-PNL-THC:CBD formulations under various conditions.

| | Initial | 2-8°C | | | RT | | | 40°C | |
|---|---|---|---|---|---|---|---|---|---|
| | | 1M | 2M | 3M | 1M | 2M | 3M | 1M | 2M |
| **PNL-THC:CBD** | 22.54 | 23.89 | 23.38 | 23.42 | 33.13 | 26.75 | 26.24 | 28.61 | 27.68 |
| | 22.51 | 28.1 | 22.73 | 23.24 | 27.86 | 27.75 | 27.38 | 30.28 | 26.60 |
| **Particle size (nm)** | 22.84 | 23.51 | 23.58 | 23.06 | | 27.22 | 27.13 | 31.54 | 26.67 |
| Mean | **22.63** | **25.17** | **23.23** | **23.24** | **30.50** | **27.24** | **26.92** | **30.14** | **26.98** |
| SD | **0.18** | **2.55** | **0.44** | **0.18** | **3.73** | **0.50** | **0.60** | **1.47** | **0.60** |
| RSD | **0.81** | **10.12** | **1.91** | **0.77** | **12.22** | **1.84** | **2.23** | **4.88** | **2.24** |

(continued)

| | Initial | 2-8°C | | | RT | | | 40°C | |
|---|---|---|---|---|---|---|---|---|---|
| | | 1M | 2M | 3M | 1M | 2M | 3M | 1M | 2M |
| P-PNL-THC:CBD Particle size (nm) | 20.2<br>21.19<br>20.94 | 22.25<br>26.37<br>24.37 | 21.59<br>21.67<br>21.97 | 21.17<br>21.24<br>21.46 | 25.3<br>31.43<br>25.74 | 24.14<br>24.77<br>24.53 | 25.43<br>24.3<br>24.71 | 27.44<br>25.68 | 24.25<br>23.78<br>23.98 |
| Mean | **20.78** | **24.33** | **21.74** | **21.29** | **27.49** | **24.48** | **24.81** | **26.56** | **24.00** |
| SD | **0.51** | **2.06** | **0.20** | **0.15** | **3.42** | **0.32** | **0.57** | **1.24** | **0.24** |
| RSD | **2.48** | **8.47** | **0.92** | **0.71** | **12.44** | **1.30** | **2.31** | **4.69** | **0.98** |

*2.4.2* For studying properties of the dispersed formulations, CBD-PNL were prepared according to the above comprising 2% CBD. CBD-PNL was diluted with HPLC grade water pre-heated to 37°C in X10, X20 and X50 dilutions. Each dilution was tested in two conditions: in 37°C and in the refrigerator (2-8°C). The solutions were evaluated for stability that included particle size determination, visual inspection/appearance, and analytical assay using HPLC.

Particle size of the 2% CBD-PNL stored as anhydrous solution was measured at initial time point and after 1 month. No difference in particles size (determined after delusion of a sample in water before measuring) was observed at 2-8°C, a slight increase in size from 19.04 to 25.97 nm at 37°C. The size was under the cut-off of <60 nm. For the diluted solutions, particle size at 37°C was larger than at 2-8°C in all three dilutions at all-time points. At 2-8°C, a trend of particle size increase in time was observed in all dilutions. At all points below 2 months, the particle size was less than 27nm. However, at 37°C the particle size increased significantly after 2 months in all dilutions. At X10 dilution, particle size at 2 months increased to 94 nm. The X10 solution showed the most significant change in size. These results support storage at room temperature or below.

Distribution of size estimated as polydispersity index (Pdi value) showed that at 2-8°C, the Pdi was below 0.3 in the diluted solutions and decreased with storage time; at 37°C Pdi was significantly smaller than observed at 2-8°C.

Analysis by visual inspection suggested that preparations X20, X50 at 37°C were in the form of clear solutions in all time points. However, preparation X10 was clear for the time course of more than 1 month; at 2 months, a turbid solution was detected. The visual inspection, together with the size and size distribution measurements reinforce the observation that increases storage temperature (probably above the melting point of tricaprine) results in instability of the nano-dispersion.

Diluted preparations kept in the refrigerator formed clear solutions at all-time points, in all three dilutions.

CBD was recovered from the diluted solutions at 37°C and 2-8°C after 1 month. The proportion of CBD recovered was compared to the concentration at initial time point. There was approximately 14% decrease in CBD recovery at 37°C. The proportion of CBD recovered from the diluted solutions at 2-8°C showed a minor decrease of about 3%.

These results demonstrate stability of the dispersed solutions while stored under 37°C and feasibility of using directly the dispersed solutions for drug administration.

### *2.5 Controlled release in pro-nanodispersion in water*

To resemble the potential use of loading this formulation into an OROS system for extended delivery of the oily formation that spontaneously form nanoparticles, base formulation and 10% cyclosporine formulation (available commercially) were loaded into ALZET pumps and placed into water pH 2 for 2 hours followed by replacing the aqueous media to pH 7 that represent the intestine. A continuous stream of lipid solution was released which formed nanoparticles. The ALZET pump can be controlled to release the content using different size and pressure applied.

## EXAMPLE 3

### *3.1 CBD relative bioavailability in animal studies*

For bioavailability studies, CBD-PNL and CBD-Advanced-PNLs (incorporating curcumin, resveratrol or piperine) were freshly prepared 30 min before each experiment, by vortex-mixing of the pre-concentrates in pre-heated to 37C$^0$ water (1:10 v/v) for 30 sec forming an O/W nano-dispersion. The obtained CBD concentration was 3mg/mL. CBD-PNL formulation and the CBD-Advanced-PNLs (CBD-Curcumin-PNL, CBD-Resveratrol-PNL and CBD-Piperine-PNL, 15 mg/kg) were administered to the animals by oral gavage (n=6). The first control group received 15 mg/kg CBD solution (propylene glycol: ethanol: water, 4.5:4.5:1) at the concentration of 3 mg/mL (n=6). Another control group received solution (propylene glycol: ethanol: water, 4.5:4.5:1) at the concentration of 3 mg/mL CBD and 2mg/ml piperine. The

administered dose of CBD was15 mg/kg, respectively the administered dose of piperine was 10 mg/kg.
100 - AM solution conc. after centrifugation
*100
AM solution conc. before centrifugation

Systemic blood samples (0.35 mL) were taken at 5 min pre-dose, 0.33, 0.66,1, 1.5, 2, 3, 5 and 6 h post-dose. To prevent dehydration equal volumes of physiological solution were administered to the rats following each withdrawal of blood sample. Plasma was separated by centrifugation (4000 g, 7 min, 4°C) and stored at -20°C pending analysis.

The plasma concentration time profiles for CBD and dispersed CBD-PNL following their oral administration to rats at a dose of 15 mg/kg are depicted in **Fig. 1.** The corresponding AUC and Cmax parameters obtained in these *in-vivo* experiments are listed in **Table 1.** Oral administration of dispersed CBD-PNL resulted in significantly increased AUC and Cmax values as compared to CBD alone (**Fig. 1** and **Table 1**).

The plasma concentration time profiles for CBD and the different CBD-Advanced-PNL formulations following their oral administration to rats at a dose of 15 mg/kg are depicted in **Figs. 2A-C.** The oral administration of CBD-curcumin-PNL resulted in significantly lower AUC and Cmax values as compared to the oral administration of CBD-PNL to rats (**Fig. 2A**). Moreover, the oral administration of CBD-curcumin-PNL resulted in similar oral bioavailability as compared to the administration of CBD alone (**Fig. 2A**). Thus, it is reasonable to assume that the incorporation of curcumin damages the proper formation of the CBD nano-lipospheres upon introduction of this pre-concentrate into water phase, i.e. the fluids of the gastrointestinal tract, resulting in poorer oral bioavailability of CBD.

The oral administration of CBD-Resveratrol-PNL resulted in increased AUC and Cmax values as compared to the free drug (**Fig. 2B**). However, the oral bioavailability of this Advanced-PNL was not superior as compared to the CBD-PNL formulation (**Fig. 2B**).

CBD-piperine-PNL oral administration resulted in a significantly increased AUC and Cmax values compared to the administration of CBD-PNL and CBD alone, suggesting advantageous bioavailability of CBD-piperine compared to other Advanced-PNL formulations incorporating curcumin or resveratrol (**Figs. 2A-C**). Thus, piperine was identified as a leading absorption enhancer and was tested in further studies investigating its effect on THC oral bioavailability.

***3.2 THC relative bioavailability in animal studies***

For bioavailability studies, dispersed THC-PNL and THC-Piperine-PNL were freshly prepared 30 min before each experiment, by vortex-mixing of the pre-concentrate in water (1:10 v/v) pre-heated to 37°C for 30 sec. The obtained THC concentration was 3 mg/mL. Dispersed THC-PNL and THC-Piperine-PNL (20 mg/kg) were administered to the animals by oral gavage (n=6). The first control group received 20 mg/kg THC dissolved in propylene glycol:ethanol (1: 1) to obtain THC concentration of 3 mg/mL (n=6). Another control group received solution (propylene glycol:ethanol, 1:1) at the concentration of 3 mg/mL THC and 2mg/ml piperine. The administered dose of THC was 20 mg/kg, respectively the administered dose of piperine was 12 mg/kg.

Systemic blood samples (0.35 mL) were taken at 5 min pre-dose, 0.33, 0.66,1, 1.5, 2, 3, 4 and 6 h post-dose. To prevent dehydration equal volumes of physiological solution were administered to the rats following each withdrawal of blood sample. Plasma was separated by centrifugation (4000 g, 7 min, 4°C) and stored at -20°C pending analysis.

Plasma concentration time profiles for THC, dispersed THC-PNL, dispersed THC-Piperine-PNL and THC with piperine in a solution following their oral administration to rats at a dose of 20 mg/kg are depicted in **Fig. 3.** The corresponding AUC and Cmax parameters obtained in these in-vivo experiments are listed in **Table 1.** Oral administration of THC with piperine resulted in similar bioavailability as compared to THC alone. The oral administration of THC-piperine-PNL resulted in a 8-fold and 4-fold increase in AUC and Cmax, respectively as compared to CBD alone and to CBD with piperine, demonstrating a 1.5-fold increase in the AUC compared to THC-PNL.

**EXAMPLE 4**

***4.1 Preliminary study of PNL-cannabinoid formulations in humans***

A preliminary clinical study in healthy volunteers receiving a single dose of a combined formulation of cannabinoids, P-PNL-THC:CBD with THC:CBD dose of approximately 20 mg each or Sativex as eight actuations suggested significantly improved bioavailability of actives in the formulation of the invention (**Figs. 4a-4b**). Similarly, subjects receiving single cannabinoids as P-PNL-THC or P-PNL-CBD with approximately 10 mg actives each, or Sativex showed a significantly better bioavailability of actives even in lower dosage forms (**Figs. 4c-4d**). This study suggested that the presently developed formulations not only enhance bioavailability of single cannabinoids, and therefore can aid in the reduction of therapeutically effective dosage, but also that this effect is not diminished in formulations comprising a combinations of cannabinoids.

***4.2 Phase 1 clinical trial of PNL-THC:CBD and P-PNL-THC:CBD***

A single-center, randomized, 5-way crossover study was conducted to compare the safety, tolerability and pharmacokinetics of PNL-cannabinoid and P-PNL-(Advanced) cannabinoid formulations administered as single doses and buccal Sativex in healthy volunteers. In brief, in both formulations an immediate rise in plasma THC and CBD was observed and both cannabinoids were detectable in the plasma 8 h post-administration. The safety profile of the tested drugs, suggests no severe and widespread drug-related adverse effects at the tested doses. The PNL and P-PNL formulations, both being self-nanoemulsifying drug delivery system, were equally effective in delivery of both CBD and THC, with a clear linear dose-plasma analyte correlation observed. Absorption of both drug substances from Sativex was consistently lower and slower when compared to the two oral formulations delivered in soft capsules.

***4.3 Clinical trial: pharmacokinetic studies***

The PNL-THC:CBD formulation yielded 1.6-fold higher peak plasma CBD levels, and within half the time, than an equivalent dose (10 mg CBD) delivered via the oromucosal spray Sativex (LSmeans of 2.8 ng/mL within 1.3 h vs. 1.8 ng/mL within 2.9 h, respectively). The overall CBD exposure following PNL-THC:CBD treatment was approximately 1.3-fold higher than following Sativex treatment (AUC of 8.7 ng/mL*h and 6.7 ng/mL*h, respectively). Relative bioavailability of the 10 mg CBD dose delivered via PNL-THC:CBD versus Sativex was 131%. A similar 1.5-fold increase in THC absorption was observed with the oral formulation, as compared to Sativex, and peaked within 1.3 h as opposed to 3.2 h following Sativex delivery. The overall extent of exposure was 1.2-fold higher (AUC of 19.3 ng/mL*h vs. 16.7 ng/mL*h for PNL-THC:CBD vs. Sativex, respectively). Bioavailability of the 10 mg THC dose delivered in the form of PNL-THC:CBD, relative to the 10 mg dose in Sativex was 116%.

Similar peak plasma CBD concentrations were measured following P-PNL-THC:CBD and Sativex treatments. When adjusting for dose differences (6.7 vs. 10 mg, respectively), P-PNL-THC:CBD enabled higher CBD absorption. Peak levels were achieved within approximately half the time when delivered in the form of P-PNL-THC:CBD versus Sativex (1.6 h vs. 2.9 h, respectively). The overall CBD exposure (AUC) following P-PNL-THC:CBD treatment was lower (5.2 ng/mL*h) as compared to oromucosal delivery (6.7 ng/mL*h). Bioavailability of the 6.7 mg CBD dose delivered in the form of P-PNL-THC:CBD, relative to the 10 mg CBD dose in Sativex was 118%. When adjusting for dose differences, the P-PNL-THC:CBD formulation was as effective as the PNL formulation in delivering THC, i.e., yielded an extrapolated 1.5-fold higher peak THC levels within half the time as compared to Sativex. As seen for the PNL formulation, overall extent of exposure were similar for both modes of delivery. Bioavailability of the 7.2 THC dose delivered in the form of P-PNL-THC:CBD, relative to the 10 mg dose in Sativex was 118%. The PK profiles of the 11-OH-THC metabolite closely mimicked those observed for THC for both routes of delivery, although elimination rates were considerably slower (~4h) for all tested drugs, as expected for the metabolite. The results of these experiments are graphically demonstrated in **Fig.5** - **Fig.7.**

Taken together, P-PNL-THC:CBD showed no superiority to the PNL-THC:CBD formulation in the kinetics in the rise of circulating CBD, THC and 11-OH-THC. The two formulations exhibited linear dose-analyte correlations: ~1.5-fold decrease in dose (from 10 to 7 mg for both THC and CBD) resulted in ~1.5 decrease in Cmax and AUC. Addition of piperine to the P-PNL-THC: CBD formulation did not result in increased THC and CBD uptake. In both formulations, an immediate rise in plasma THC and CBD was observed and both cannabinoids were detectable in the plasma 8 h post-administration.

***4.3 Clinical trial: bioavailability studies***

When comparing bioavailability of the active ingredient upon oral vs. oromucosal delivery, all tested oral formulations showed enhanced CBD and/or THC bioavailability (**Table 5**). Relative bioavailability of 10 mg CBD formulation as compared to Sativex was estimated at 131% with the PNL platform, and 118% with the P-PNL. THC biovailability was also higher upon oral delivery, and was similar for both the PNL and P-PNL formulations (116% and 118% in comparison to Sativex, respectively).

**Table 5:** Bioavailablity of CBD and THC upon oral delivery, in comparison to Sativex (PK Population).

| Substance | PK parameter (ng/mL h) | Treatment | Bioavailability vs. Sativex | 90% CI |
|---|---|---|---|---|
| **CBD** | **AUC(0-t)** | **PNL-THC:CBD** | 131.2% | [111.0%; 155.1%] |
| | | **P-PNL-THC: CBD** | 117.6% | [99.6%; 138.9%] |

(continued)

| Substance | PK parameter (ng/mL h) | Treatment | **Bioavailability** vs. **Sativex** | 90% CI |
|---|---|---|---|---|
| **THC** | **AUC(0-t)** | **PNL-THC:CBD** | 115.5% | [102.6%; 130.2%] |
| | | **P-PNL-THC: CBD** | 118.4% | [105.1%; 133.3%] |

***4.4 Safety and tolerability results***

A total of 24 AEs were reported, with a similar incidence across investigational drug treatment sessions. Out of the 15 subjects, 8 (53.3%) suffered from at least one AE. In each one of the 5 treatments, between 3 and 4 subjects suffered from at least one AE. None were serious, 3 of them were considered moderate and the rest mild, 58.3% (14/24) were considered related to the study drugs. Headache was the most frequently reported AE (13/24, 54.2%, reported by 6 (out of the 15), while all other events were reported up to twice throughout the study. The majority of AEs were mild (87.5%), and considered related to the study treatment. The remaining AEs were rated moderate; none were serious. Of the ten AEs considered possibly related to the study drug, 8 were reported by two subjects, with one subject suffering from a headache after all five test sessions and another subject reporting headache after 3/5 sessions.

No clinically significant laboratory test abnormalities were noted in the end of study blood and urine samples. Similarly, no clinically significant abnormalities in vital signs, ECG recordings or physical findings were noted.

## Claims

1. A formulation for use in oral administering of an effective amount of a cannabinoid composition comprising a combination of Tetrahydrocannabinol (THC) and Cannabidiol (CBD), or an isoform, a derivative, a precursor, or a metabolite thereof selected from $\Delta^9$-THC, $\Delta^8$-THC, an acid form of THC or CBD, namely THC-A or CBD-A, 11-OH-THC and THC-11-oic acid forms, in pharmaceutically acceptable carrier or excipient, the cannabinoid composition comprising between 0.2 and 15wt% of THC and CBD of the total weight of the formulation, the formulation further comprising

   **(a)** at least one surfactant,
   **(b)** at least one lipid component,
   **(c)** a water soluble biocompatible amphiphilic solvent,

   wherein the formulation is free of piperine; and
   wherein upon administering and contact with an aqueous medium, the formulation is converted into particles of a size of less than about 500 nm, thereby increasing bioavailability of the administered cannabinoid composition.

2. The formulation according to claim 1, wherein said any of THC and CBD or an isoform, a derivative, a precursor, or a metabolite thereof that is a synthetic cannabinoid or a natural cannabinoid obtained from cannabis extracts as single cannabinoid or a combination of cannabinoids.

3. The formulation according to claim 1, wherein the ratio THC:CBD is about 1:1.

4. The formulation according to claim 1, wherein the THC is in excess.

5. The formulation according to claim 1, wherein the CBD is in excess.

6. The formulation according to claim 1, further comprising at least one additional therapeutically active or non-active agent, or a combination thereof, excluding piperine.

7. The formulation according to claim 6, wherein the therapeutically active agent is selected from analgesics, anti-rheumatic, antibiotics, chemotherapeutic drugs, immune-suppressants, antipsychotics, drugs for neurological disorders, drugs for the treatment of AIDS, and combinations thereof.

## Patentansprüche

1. Formulierung zur Verwendung bei der oralen Verabreichung einer wirksamen Menge einer Cannabinoidzusammensetzung, die eine Kombination aus Tetrahydrocannabinol (THC) und Cannabidiol (CBD) umfasst, oder einer Isoform, eines Derivats, einer Vorstufe oder eines Metaboliten davon, ausgewählt aus $\Delta^9$-THC, $\Delta^8$-THC, einer Säureform von THC oder CBD, insbesondere THC-A oder CBD-A, 11-OH-THC- und THC-11-Säureformen, in einem pharmazeutisch verträglichen Träger oder Hilfsstoff, wobei die Cannabinoidzusammensetzung zwischen 0,2 und 15 Gew.-% THC und CBD bezogen auf das Gesamtgewicht der Formulierung umfasst, wobei die Formulierung ferner Folgendes umfasst:

   (a) mindestens ein Tensid,
   (b) mindestens eine Lipidkomponente,
   (c) ein wasserlösliches biokompatibles amphiphiles Lösungsmittel,
   wobei die Formulierung kein Piperin enthält; und
   wobei die Formulierung bei Verabreichung und Kontakt mit einem wässrigen Medium in Partikel einer Größe von weniger als etwa 500 nm umgewandelt wird, wodurch die Bioverfügbarkeit der verabreichten Cannabinoidzusammensetzung erhöht wird.

2. Formulierung nach Anspruch 1, wobei jedes des THC und des CBD oder einer Isoform, eines Derivats, einer Vorstufe oder eines Metaboliten davon ein synthetisches Cannabinoid oder ein natürliches Cannabinoid ist, das als einzelnes Cannabinoid oder eine Kombination von Cannabinoiden aus Cannabisextrakten gewonnen wird.

3. Formulierung nach Anspruch 1, wobei das Verhältnis THC:CBD etwa 1:1 beträgt.

4. Formulierung nach Anspruch 1, wobei das THC in größerer Menge vorliegt.

5. Formulierung nach Anspruch 1, wobei das CBD in größerer Menge vorliegt.

6. Formulierung nach Anspruch 1, die ferner mindestens einen zusätzlichen therapeutisch aktiven oder nicht aktiven Wirkstoff umfasst oder eine Kombination davon, die nicht Piperin enthält.

7. Formulierung nach Anspruch 6, wobei der therapeutisch aktive Wirkstoff aus Analgetika, Antirheumatika, Antibiotika, Chemotherapeutika, Immunsuppressiva, Antipsychotika, Medikamenten für neurologische Erkrankungen, Medikamenten zur Behandlung von AIDS und Kombinationen davon ausgewählt ist.

## Revendications

1. Formulation destinée à être utilisée dans l'administration orale d'une quantité efficace d'une composition de cannabinoïdes comprenant une combinaison de tétrahydrocannabinol (THC) et de cannabidiol (CBD), ou une isoforme, un dérivé, un précurseur ou un métabolite de celui-ci choisi parmi $\Delta^9$-THC, $\Delta^8$-THC, une forme acide de THC ou de CBD, à savoir les formes acide THC-A ou CBD-A, 11-OH-THC et THC-11-oïque, dans un support ou excipient pharmaceutique ment acceptable, la composition de cannabinoïdes comprenant entre 0,2 et 15 % en poids de THC et de CBD du poids total de la formulation, la formulation comprenant en outre :

   (a) au moins un tensioactif,
   (b) au moins un composant lipidique,
   (c) un solvant amphiphile biocompatible soluble dans l'eau, dans laquelle la formulation est exempte de pipérine ; et dans laquelle lors de l'administration et du contact avec un milieu aqueux, la formulation est convertie en particules d'une taille inférieure à environ 500 nm, augmentant ainsi la biodisponibilité de la composition de cannabinoïdes administrée.

2. Formulation selon la revendication 1, dans laquelle ledit THC et CBD ou une isoforme, un dérivé, un précurseur ou un métabolite de celui-ci qui est un cannabinoïde synthétique ou un cannabinoïde naturel obtenu à partir d'extraits de cannabis sous forme de cannabinoïde unique ou d'une combinaison de cannabinoïdes.

3. Formulation selon la revendication 1, dans laquelle le rapport THC:CBD est d'environ 1:1.

**4.** Formulation selon la revendication 1, dans laquelle le THC est en excès.

**5.** Formulation selon la revendication 1, dans laquelle le CBD est en excès.

**6.** Formulation selon la revendication 1, comprenant en outre au moins un agent thérapeutiquement actif ou non actif supplémentaire, ou une combinaison de ceux-ci, à l'exclusion de la pipérine.

**7.** Formulation selon la revendication 6, dans laquelle l'agent thérapeutiquement actif est choisi parmi les analgésiques, les antirhumatismaux, les antibiotiques, les médicaments chimiothérapeutiques, les immunosuppresseurs, les antipsychotiques, les médicaments pour les troubles neurologiques, les médicaments pour le traitement du SIDA et leurs combinaisons.

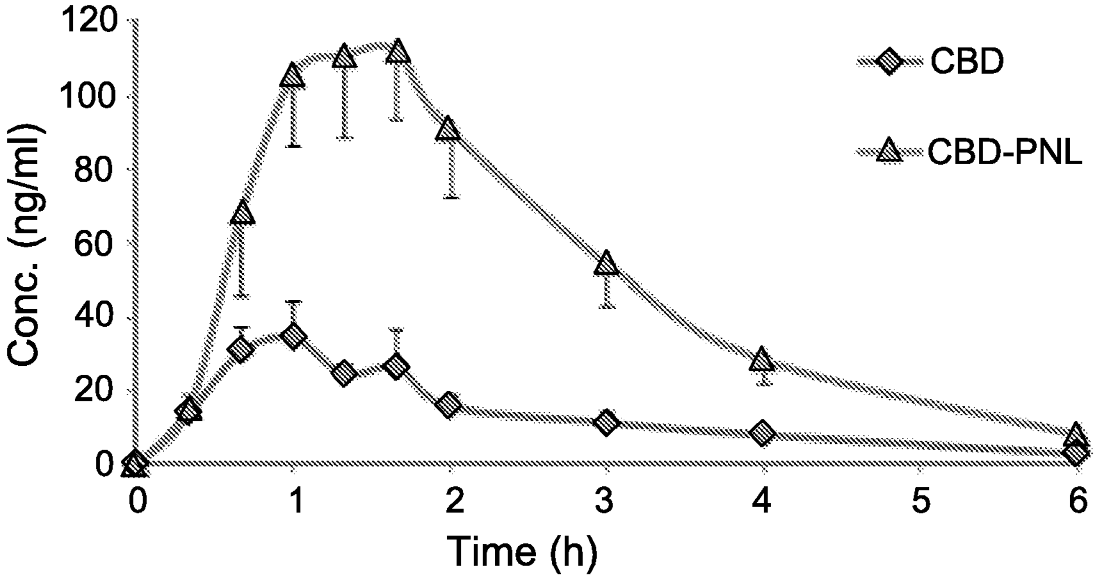


Fig. 1

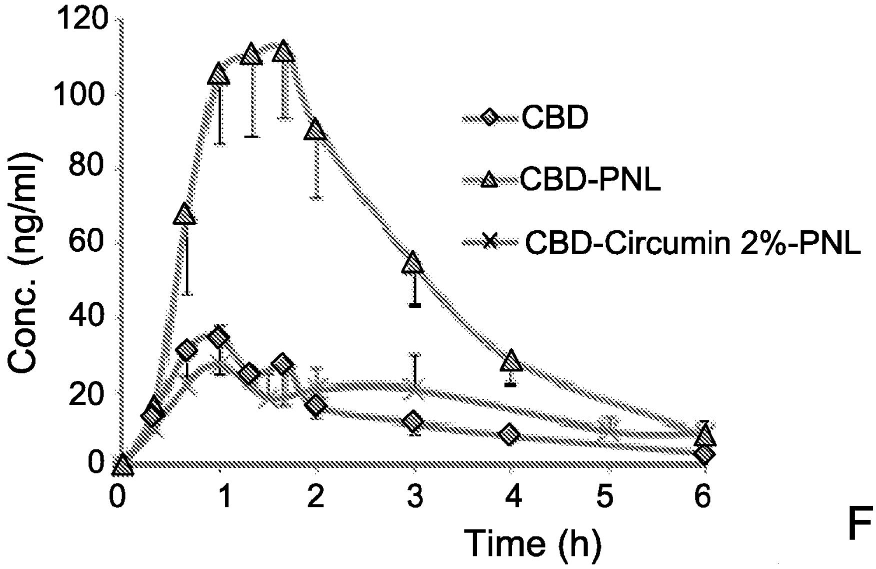


Fig. 2A

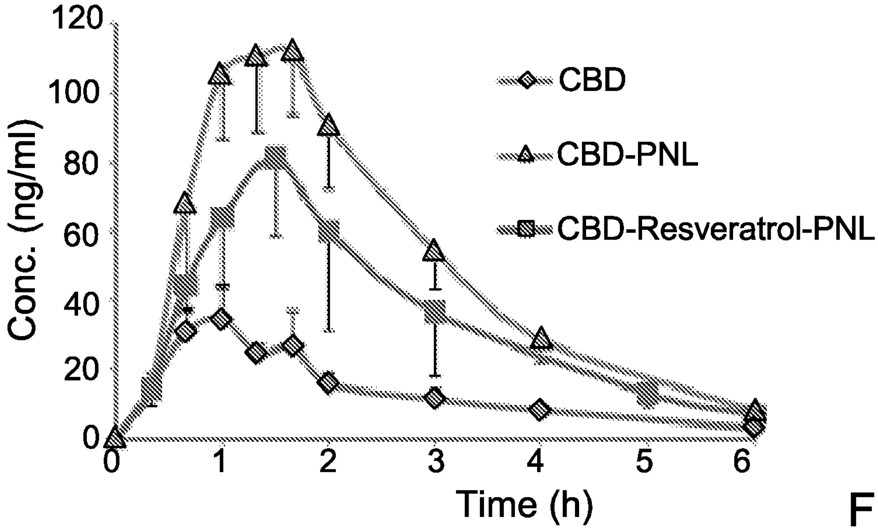


Fig. 2B

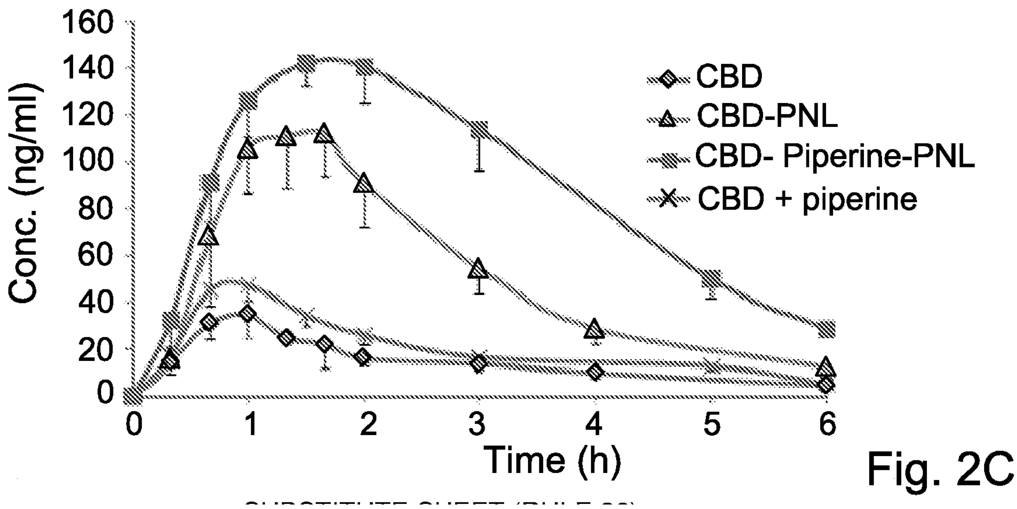


Fig. 2C

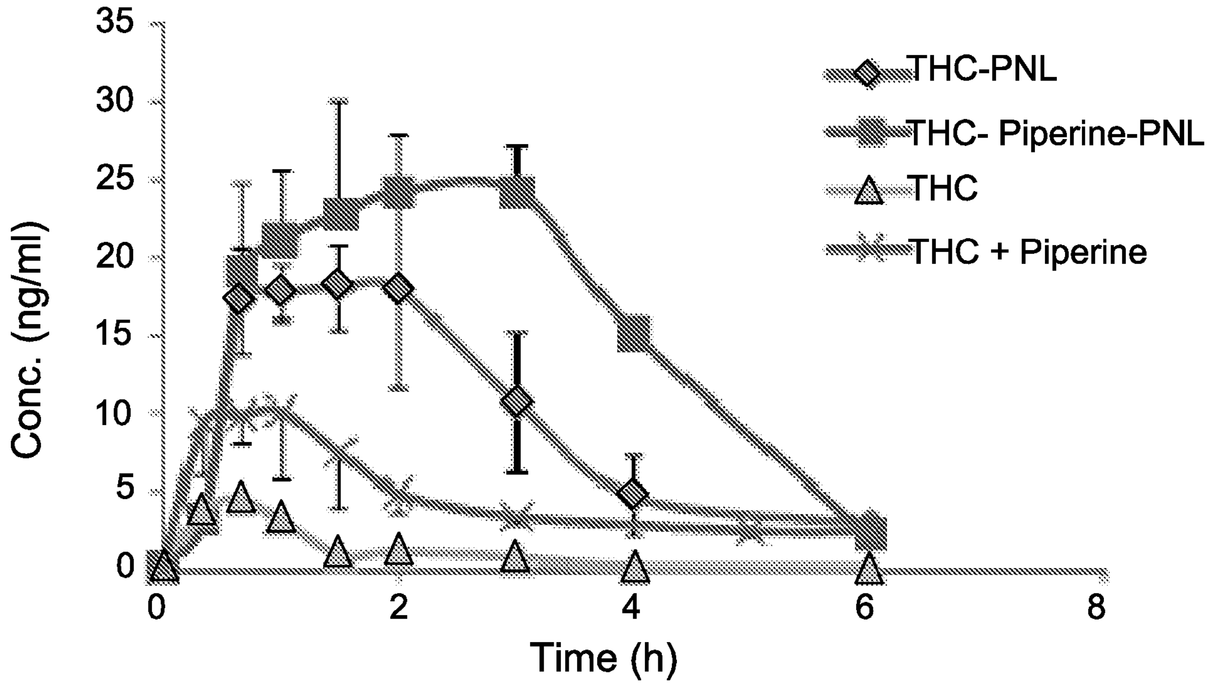
35
30
25
20
15
10
5
0
Conc. (ng/ml)
0
2
4
6
8
Time (h)
THC-PNL
THC- Piperine-PNL
THC
THC + Piperine

Fig. 3

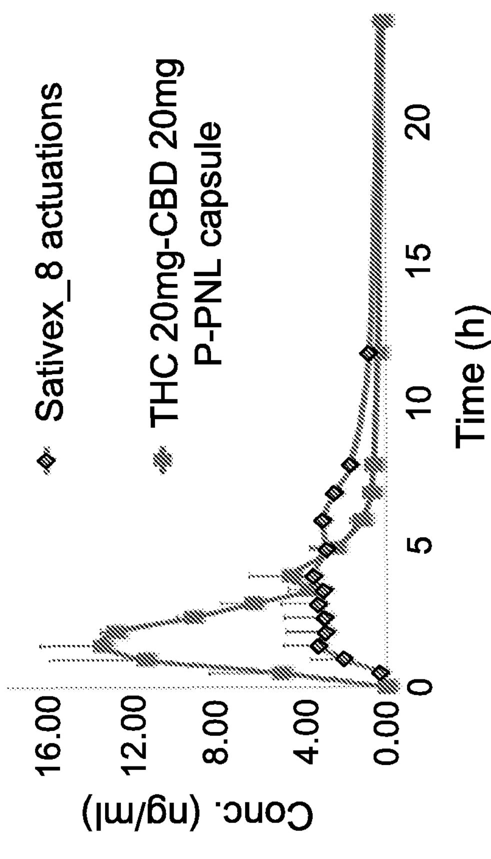


Fig. 4A

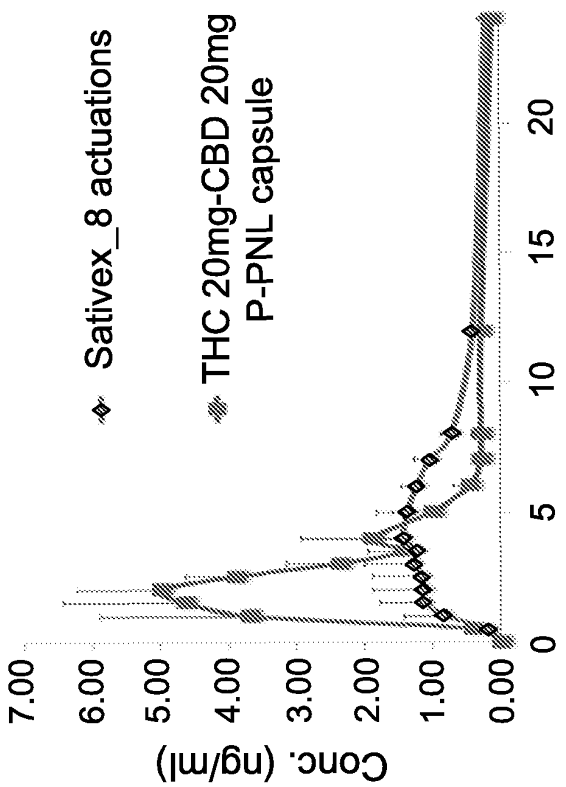


Fig. 4B

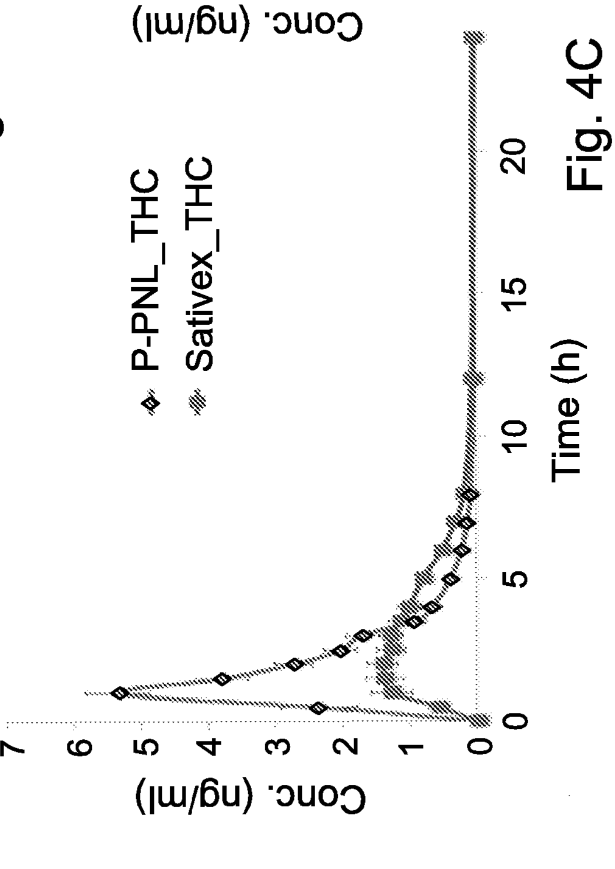


Fig. 4C

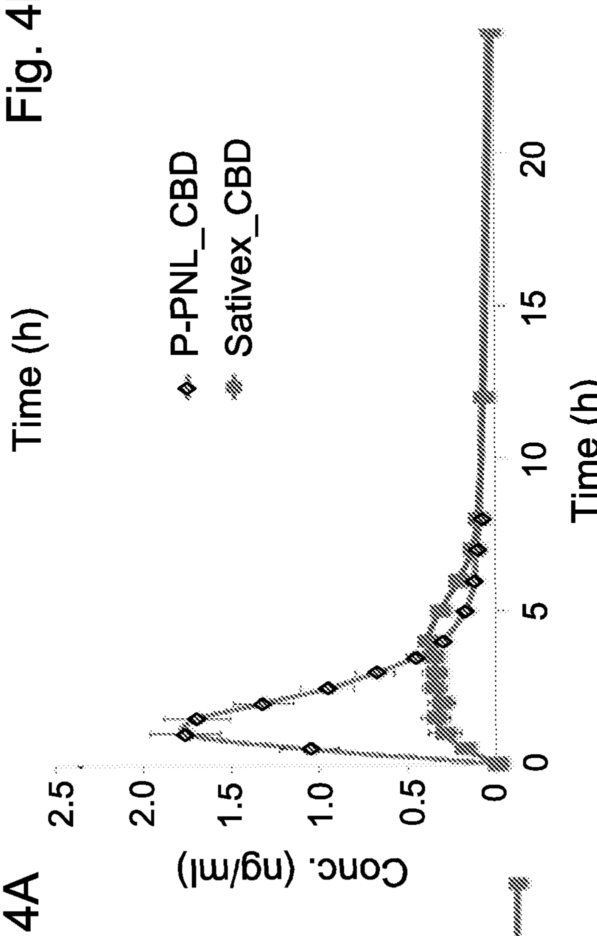


Fig. 4D

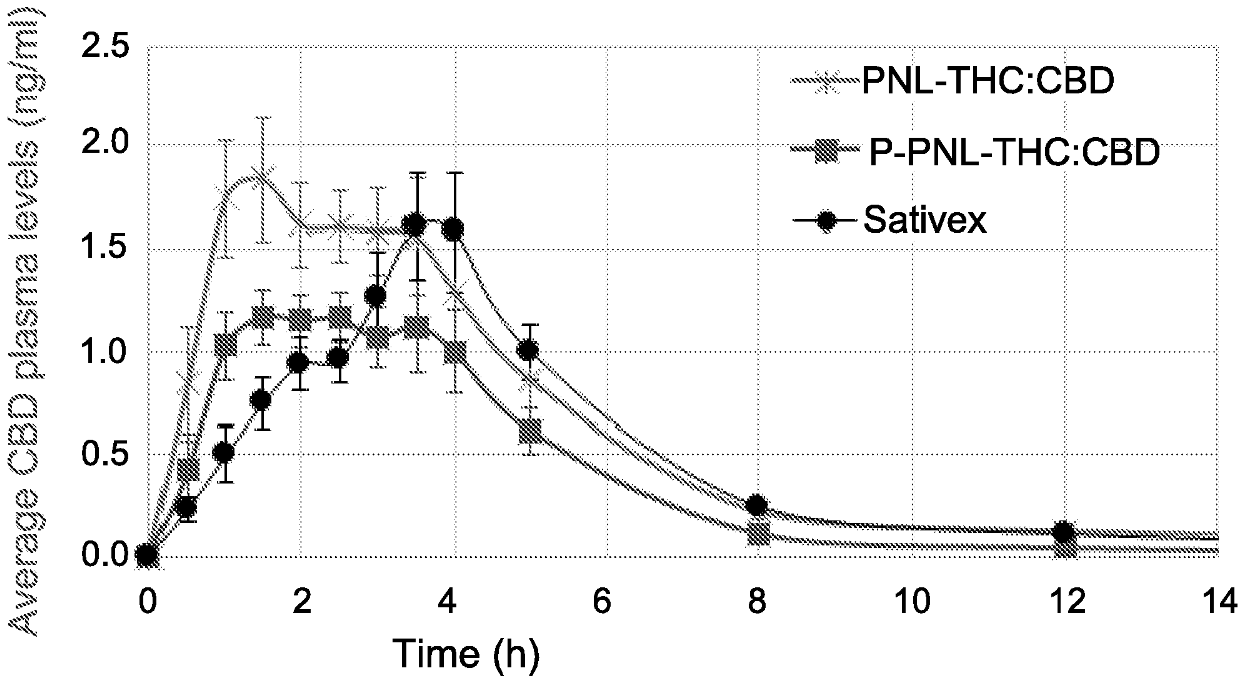
Average CBD plasma levels (ng/ml)
2.5
2.0
1.5
1.0
0.5
0.0
0
2
4
6
8
10
12
14
Time (h)
PNL-THC:CBD
P-PNL-THC:CBD
Sativex

Fig. 5

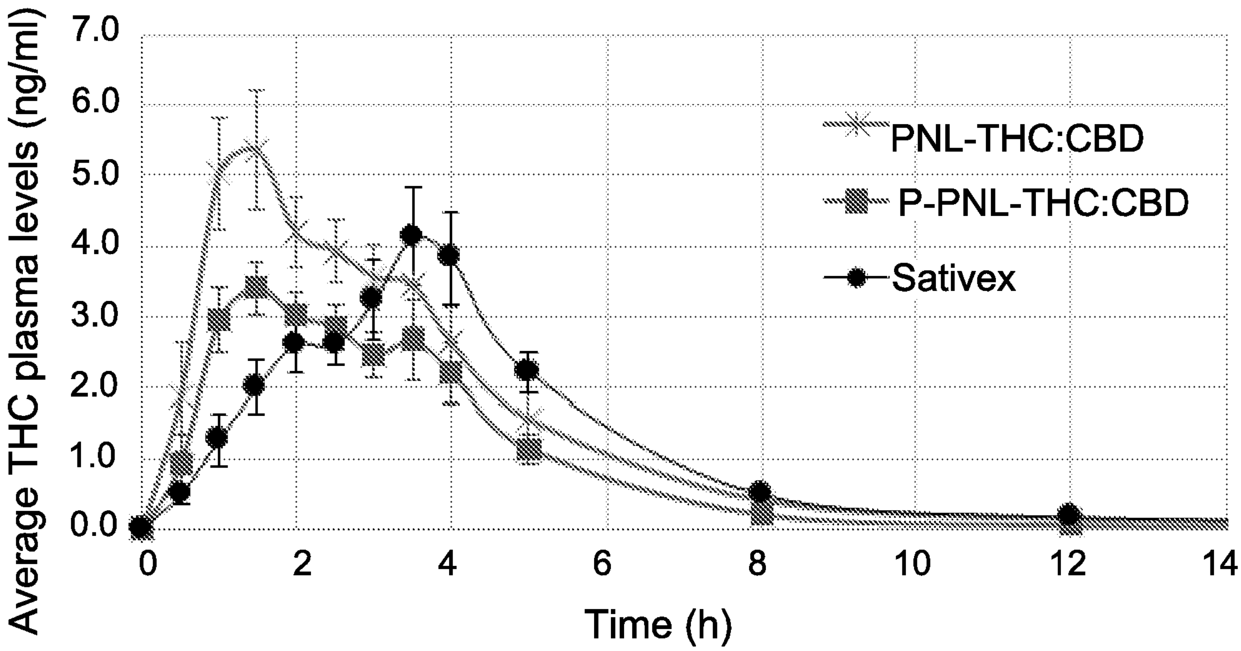
Average THC plasma levels (ng/ml)
7.0
6.0
5.0
4.0
3.0
2.0
1.0
0.0
0
2
4
6
8
10
12
14
Time (h)
PNL-THC:CBD
P-PNL-THC:CBD
Sativex

Fig. 6

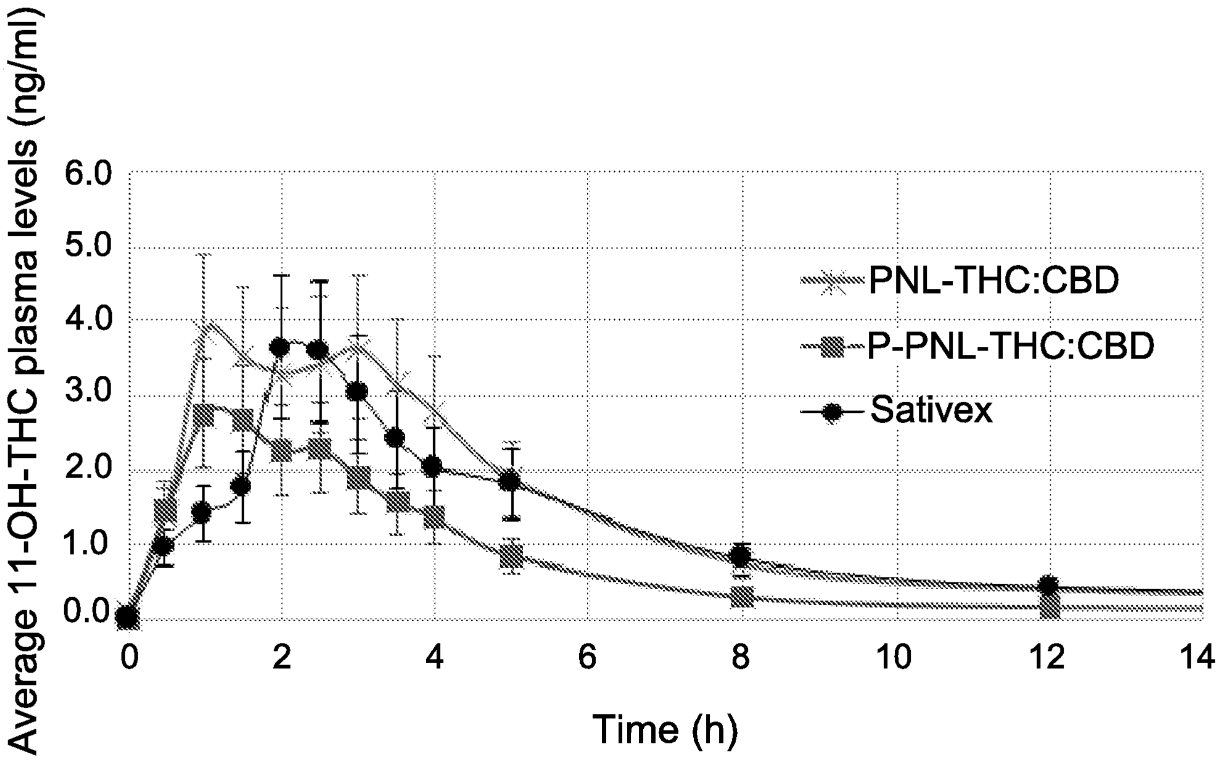

Average 11-OH-THC plasma levels (ng/ml)
6.0
5.0
4.0
3.0
2.0
1.0
0.0
0
2
4
6
8
10
12
14
Time (h)
PNL-THC:CBD
P-PNL-THC:CBD
Sativex


Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7919113 B
- WO 07056242 A
- WO 13108254 A